# EUROPÄISCHE PATENTSCHRIFT

(11) **EP 2 559 725 B1**
(45) Veröffentlichungstag und Bekanntmachung des Hinweises auf die Patenterteilung: **08.05.2024**
(21) Anmeldenummer: 11006825.1
(22) Anmeldetag: 19.08.2011
(51) Int. Cl.: C08J 11/10, C08J 11/24, B01J 19/00, B01J 19/20, C07C 51/09, C07C 59/08

(54) **Verfahren und Vorrichtung zur Rückgewinnung von Lactid aus Polylactid bzw. Glycolid aus Polyglycolid**
Method and device for recovery of lactide from polylactide and glycolide from polyglycolide
Procédé et dispositif de récupération de lactide à partir de polylactide ou de polylactile

(43) Veröffentlichungstag der Anmeldung: 20.02.2013
(73) Patentinhaber: Uhde Inventa-Fischer GmbH, 13509 Berlin (DE)
(72) Erfinder: Hagen, Rainer, 13465 Berlin (DE)
(74) Vertreter: Pfenning, Meinig & Partner mbB

(56) Entgegenhaltungen:
- EP-A1- 1 980 581
- WO-A1-93/02075
- WO-A1-2011/029648
- JP-A- 7 309 863
- JP-A- H07 309 863
- JP-A- H10 130 256
- JP-A- 2007 023 176
- JP-A- 2009 249 508
- US-A- 5 229 528

## Beschreibung

Die vorliegende Erfindung betrifft ein Verfahren zur Rückgewinnung von Lactid aus Polylactid (PLA) oder Glycolid aus Polyglycolid (PGA), bei dem in einem ersten Schritt PLA bzw. PGA mit einem hydrolysierenden Medium in Kontakt gebracht und hydrolytisch zu Oligomeren abgebaut wird. In einem weiteren Schritt erfolgt eine zyklisierende Depolymerisation der im ersten Schritt erhaltenen Oligomeren zum Lactid bzw. Glycolid. Zudem betrifft die vorliegende Erfindung eine Vorrichtung, die auf der Kombination einer Hydrolysevorrichtung mit einem Depolymerisationsreaktor beruht, mit dem das zuvor beschriebene Verfahren durchgeführt werden kann. Kern des erfindungsgemäßen Verfahrens ist eine partielle Hydrolyse der ursprünglich eingesetzten polymeren Materialien in Kombination mit einer zyklisierenden Depolymerisation.

Es ist bekannt, PLA durch vollständige Hydrolyse mit Wasser oder wasserhaltiger Milchsäure in Milchsäure zu spalten, die nach entsprechender Reinigung auf bekannten Wegen wieder zu PLA umgewandelt werden kann.

Diese Methoden haben den Nachteil, dass während der vollständigen Hydrolyse partielle Racemisierung eintritt. Dabei entsteht aus PLA, das beispielsweise zu 98 % aus L-Milchsäureeinheiten und zu 2 % aus D-Einheiten besteht, Milchsäure mit einem D-Anteil, der deutlich größer ist als die zu erwartenden 2 %.

M. Faisal et al. (Asian Journal of Chemistry Vol. 19, No. 3 (2007), S. 1714-1722) untersuchten die Hydrolyse von PLA im Temperaturbereich zwischen 160-350°C über 3 h. Hohe Ausbeuten an Milchsäure erhält man bei hohen Temperaturen oder langen Verweilzeiten, Bedingungen, bei denen auch die L-Milchsäure teilweise zu D-Milchsäure racemisiert.

M. Yagihashi und T. Funazukuri (Ind. Eng. Chem. Res. 2010, 49, S. 1247-1251) benutzten verdünnte wässrige Natronlauge zur Hydrolyse von PLA im Temperaturbereich zwischen 70-180 °C und verglichen mit reinem Wasser. Bei Reaktionszeiten im Bereich von 20-60 min wurde keine Racemisierung beobachtet. Bei der Umsetzung in ein technisches Verfahren muss der Natriumgehalt der erzeugten Milchsäure mit hohem Aufwand entfernt werden.

Ein weiterer Nachteil aller Verfahren, die mit vollständiger Hydrolyse des PLA zu Milchsäure arbeiten, gegenüber dem erfindungsgemäßen Verfahren ist, dass die gereinigte, so rezyklierte Milchsäure in einem Prozess zur Herstellung von PLA aus Milchsäure, bestehend aus Entwässerung, Polykondensation zum Oligomer, Depolymerisation zum Lactid, Lactidreinigung, Ringöffnungspolymerisation des Lactids zum PLA, das im industriellen Maßstab fast ausschließlich benutzt wird, alle genannten Prozessschritte durchlaufen muss. Entwässerung und Polykondensation verbrauchen Energie zur Abtrennung von Wasser, erhöhen durch partielle Racemisierung den D-Anteil im Polykondensat der L-Milchsäure und benötigen Apparatekapazität zur Durchführung.

Aus DE 196 37 404 B4 ist ein Verfahren bekannt, das PLA ohne vorhergehende Hydrolyse durch Depolymerisation in Dilactid umsetzt. Nachteil ist die mit 3-7 % sehr hohe Katalysatormenge und eine mit bis zu 300°C sehr hohe Temperatur. Die Depolymerisation von hochmolekularem PLA zu Lactid ist wegen der niedrigen Endgruppenkonzentration sehr langsam verglichen mit PLA-Oligomer, so dass die Reaktionsgeschwindigkeit durch hohe Katalysatorkonzentration und hohe Temperatur erhöht werden muss.

In der WO 2010/118954 ist ein Verfahren beschrieben, das PLA-haltige Polymerblends in einem Lösungsmittel auflöst, die ungelösten Polymeranteile entfernt, das PLA hydrolytisch zu Milchsäure oder einem Derivat davon depolymerisiert und das Produkt reinigt. Nachteilig bei diesem Verfahren ist das Lösungsmittel, das aus dem Produkt entfernt und zurückgewonnen werden muss.

WO 2011/029648 beansprucht ein Verfahren zum Rezyklieren einer Mischung von PLA unterschiedlichen Gehalts an optischen Isomeren, das mit einer Trennung dieser Isomere auf der Stufe des Lactids verknüpft ist. In diesem Verfahren wird das PLA in einem Lösungsmittel gelöst und anschließend durch Umesterung depolymerisiert. Das Umesterungsprodukt wird vom Lösungsmittel getrennt und dann durch zyklisierende Depolymerisation zu Rohlactid umgesetzt. Die Reinigung des Rohlactids schließt eine Abtrennung des meso-Lactids ein. Nachteilig ist bei diesem Verfahren wiederum das Lösungsmittel, das abgetrennt und zurückgewonnen werden muss. Die Verweilzeit in der Depolymerisation ist sehr lange.

Die japanische Patentanmeldung JP 2009-249508 beschreibt ein Recycling-Verfahren, das partielle Hydrolyse mit Depolymerisation des entstandenen Oligomers zum Lactid kombiniert. Es hat den Vorteil, die Racemisierung während der Hydrolyse zu verringern. Nachteilig ist, dass die Hydrolyse im diskontinuierlichen Verfahren mit Wasserdampf im festen PLA geschieht. Auf diese Weise ist ein großtechnischer Prozess schwer wirtschaftlich zu realisieren. JP H07 309863 A beansprucht ein Verfahren zur Gewinnung von Lactid durch Schmelzmischen von Polylactid (PLA) in Gegenwart von Wasser und einem Katalysator bei einer Temperatur von 200 bis 400 °C in einem Extruder, wobei das zugesetzte Polylactid geschmolzen und hydrolytisch zu Oligomeren depolymerisiert wird, die in einem weiteren Schritt zum Lactid cyclisch depolymerisiert werden.

Es besteht deshalb Bedarf an einem kontinuierlichen Recycling-Verfahren für PLA, das den Aufwand an Energie und Apparaten verringert und gleichzeitig die partielle Racemisierung senkt.

Somit ist es Aufgabe der vorliegenden Erfindung, ein im Vergleich zu den zuvor beschriebenen Verfahren verbessertes Rückgewinnungsverfahren für Lactid bzw. Glycolid aus ihren jeweiligen Polymeren anzugeben, sowie eine entsprechende Vorrichtung zur Durchführung dieses Verfahrens.

Diese Aufgabe wird bezüglich des Verfahrens mit den Merkmalen des Patentanspruchs 1 sowie hinsichtlich der Vorrichtung mit den Merkmalen des Patentanspruchs 15 gelöst. Die jeweiligen abhängigen Patentansprüche stellen dabei vorteilhafte Weiterbildungen dar.

Erfindungsgemäß wird somit ein Verfahren zur Rückgewinnung von Lactid aus Polylactid (PLA) oder Glycolid aus Polyglycolid (PGA) angegeben, bei dem
a) in einer ersten Stufe oder einem ersten Schritt PLA bzw. PGA in einem Extruder oder einem Schmelzrost aufgeschmolzen und in der Schmelze mit einem hydrolysierenden Medium in Kontakt gebracht und hydrolytisch zu PLA-Oligomeren mit einer zahlengemittelten Molmasse Mₙ zwischen 162 und 10.000 g/mol (gemessen durch Säure-Base-Titration der Carboxylgruppen) bzw. zu PGA-Oligomeren mit einer zahlengemittelten Molmasse Mₙ zwischen 134 und 10.000 g/mol (gemessen durch Säure-Base-Titration der Carboxylgruppen) abgebaut werden, wobei der hydrolytische Abbau in einer Hydrolysevorrichtung (2), die eine beheizbare Rohrstrecke aufweist, durchgeführt wird und
b) in einer weiteren Stufe die PLA- bzw. PGA-Oligomeren anschließend einer zyklisierenden Depolymerisation zu Lactid bzw. Glycolid unterworfen werden, die in einem stromabwärts der Hydrolysevorrichtung (2) angeordneten Depolymerisationsreaktor (9), der als Umlaufverdampfer, Fallfilmverdampfer, Dünnschichtverdampfer oder als eine Kombination aus zwei oder drei dieser Bauarten ausgeführt ist, durchgeführt wird, wobei vor der zyklisierenden Depolymerisation in Stufe b) den Oligomeren ein Katalysator zugegeben wird.

Vergleicht man den erfindungsgemäßen Prozess mit bekannten Recycling-Prozessen des PLA, die mit vollständiger Hydrolyse der Abfälle zur Milchsäure arbeiten, so sind folgende Vorteile zu nennen:
- Das partielle Hydrolysat nach dem erfindungsgemäßen Verfahren muss nicht mehr durch Polykondensation zum Oligomer aufgebaut werden wie Milchsäure aus einer vollständigen Hydrolysereaktion. Das spart Energie, die zur Vakuumverdampfung des chemisch während der Polykondensation gebildeten Wassers nötig ist.
- Zugleich erfährt das partielle Hydrolysat nicht die Racemisierung, die nicht nur beim Aufbau des Oligomers aus Milchsäure beobachtet wird, sondern auch bei der vollständigen Hydrolyse des PLA zur Milchsäure. Da die PLA-Qualität durch Racemisierung gemindert ist, ist das ein Qualitätsvorteil oder - bei vorhandenen Trennmöglichkeiten zwischen L- und D-Milchsäure oder zwischen L-, D-, und meso-Lactid - eine Ersparnis an apparativem Aufwand und Energie zur Trennung dieser Komponenten.
- Das erfindungsgemäße Verfahren bietet insbesondere an mehreren Stellen die Möglichkeit zur Abtrennung von Fremdstoffen, die im geschmolzenen PLA als Feststoffe vorliegen können und vor der Hydrolyse durch ein Schmelzefilter zurückgehalten werden können:
   o Nach der Hydrolyse zum Oligomer liegt eine dünnflüssige Schmelze vor, aus der mitgeführte feste Fremdstoffe und deren feste Zersetzungsprodukte durch Filtration wenig aufwändig abgetrennt werden können. Hierfür eignet sich z.B. ein Wechselfilter mit Drahtgewebe oder Vlies, bei dem die beladenen Filterronden ohne Unterbrechung des Schmelzestroms durch frische ersetzt und der Filtervorrichtung entnommen werden können. Geeignet sind auch Filtervorrichtungen, bei denen das Filtermedium durch Rückspülung regeneriert werden kann. Die Filtration nach der Hydrolyse ist eine Option. Sie kann je nach Art der in den Abfällen vorhandenen Verunreinigungen wahrgenommen oder weggelassen werden.
   ∘ Die zweite Möglichkeit zur Abtrennung von Fremdstoffen bietet die zyklisierende Depolymerisation des Hydrolysats. Dort werden > 95 % des Hydrolysats in Lactid umgewandelt, das im Vakuum verdampft und den Reaktor verlässt. Zurück bleibt - wie auch im Herstellprozess des frischen PLAs - ein oligomerer Rückstand, der neben den Produkten des thermischen Oligomerabbaus auch die schwerflüchtigen oder festen Fremdstoffe bzw. deren Zersetzungsprodukte enthält. Dieser Rückstand verlässt den Prozess und dient als natürlicher Auslass für Verunreinigungen.
   ∘ Alle Verunreinigungen, die flüchtiger sind als das Lactid, z.B. entstanden durch thermische Zersetzung von Fremdstoffen, bleiben bei der nachfolgenden Lactid-Kondensation in der Dampfphase und werden zusammen mit den PLA-typischen Nebenprodukten, wie Wasser und Milchsäure, durch die Vakuumpumpen abgesaugt und verlassen so den Prozess. Reste, die eventuell im Rohlactid verbleiben, werden in der nachfolgenden Lactidreinigung entfernt.
   Fremdstoffe oder deren Zersetzungsprodukte, die mit dem Rohlactid reagieren, müssen vor der zyklisierenden Depolymerisation entfernt werden, entweder in der Vorreinigung der Abfälle oder durch Filtration nach der Hydrolyse.

In den Ansprüchen sowie in der folgenden Beschreibung werden unter den jeweiligen Begriffen die folgenden Definitionen verstanden:
*Lactid:* zyklischer Ester aus 2 Milchsäuremolekülen, der in der Form des reinen L-Lactids (S,S-Lactid), D-Lactids (R,R-Lactids) oder meso-Lactids (S,R-Lactid) vorkommen kann oder (in den meisten Fällen) in der Form eines Gemisches aus mindestens zwei dieser Komponenten. Die zyklisierende Depolymerisation erzeugt *Rohlactid,* das neben den genannten Isomeren des Lactids noch lineare Oligomere, höhere zyklische Oligomere und Reste von Milchsäure und Wasser enthalten kann. Unter L-Lactid ist der zyklische Ester aus zwei L-Milchsäureeinheiten zu verstehen, unter D-Lactid der zyklische Ester aus zwei D-Milchsäureeinheiten, unter meso-Lactid der zyklische Ester aus einer D- und einer L-Milchsäureeinheit. *Racemisches Lactid* (rac-Lactid) ist ein 1:1-Gemisch aus L- und D-Lactid.
*Glycolid:* zyklischer Ester aus zwei Glycolsäuremolekülen;
*Polylactid (PLA):* Polymer aus Milchsäureeinheiten, z.B. hergestellt durch ringöffnende Polymerisation des L-, D- oder meso-Lactids oder einer Mischung aus zwei oder drei dieser Lactide. Es kann sich auch um eine Mischung aus Polymeren aus den genannten reinen oder gemischten Lactiden handeln. Unter (reinem) PLA sei hier auch PLA verstanden, das Stereokomplex-Kristallite enthält oder ganz daraus besteht. PLA weist in der Regel ein zahlengemitteltes Molekulargewicht > 10.000 g/mol auf.
*Polyglycolid (PGA):* Polymer aus Glycolsäureeinheiten, z.B. hergestellt durch ringöffnende Polymerisation des Glycolids. PGA weist in der Regel ein zahlengemitteltes Molekulargewicht > 10.000 g/mol auf.
*PLA-Abfälle bzw. PGA-Abfälle:* Die Erfindung eignet sich besonders zum Rezyklieren von PLA- oder PGA-Abfällen, die aus der PLA-oder PGA-Verarbeitung kommen, z.B. abgeschnittene Randstreifen von PLA- oder PGA-Folien oder Angüsse aus Spritzgussteilen. Diese Abfälle sind normalerweise sortenrein, d.h. sie enthalten keine anderen Kunststoffe. Die Erfindung eignet sich jedoch auch für gebrauchte Verpackungen, Textilien, Bauteile aus technischen Kunststoffen aus sortenreinem PLA oder PGA, das mit Fremdstoffen aus dem Gebrauch verunreinigt ist, die durch vorangehende mechanische Trennmethoden nicht vollständig abgetrennt werden können. Beispiele für derartige Fremdstoffe sind Zucker aus zuckerhaltigen Getränken oder Speiseöle, die durch Migration in die Flaschenwand eingedrungen sind und durch Wäsche der gebrauchten Flasche nicht vollständig entfernt werden können.

Schließlich eignet sich die Erfindung für PLA- oder PGA-Abfälle, die durch vorangehende mechanische Trennmethoden, wie Sortieren, Waschen, Flottieren, Zerkleinern, Sichten aus einer Mischung oder einem Verbund mit anderen Stoffen, z.B. Papier, Metall, Glas, anderen Kunststoffen gewonnen wurden und noch Reste dieser Fremdstoffe enthalten. Beispiele sind Textilien aus gemischten Fasern oder aus Mehrkomponenten-Fäden, Mehrschichtfolien mit einer oder mehreren Schichten aus PLA, PGA oder PLA- oder PGA-haltigen Kompositen. Ein weiteres Beispiel ist die Fremdpolymer-Fraktion, die nach Zerkleinerung und Sortierung von gebrauchten Kunststoffflaschen entsteht. Eine vollständige mechanische Trennung von Fremdstoffen ist hier aus wirtschaftlichen Gründen normalerweise nicht möglich.

Reste von Fremdstoffen stellen für das erfindungsgemäße Verfahren unter bestimmten Voraussetzungen kein Hindernis dar. Fremdstoffe stammen aus der Verarbeitung, dem Gebrauch oder der Entsorgung der Verpackungen, Textilien oder technischen Kunststoffe. Dabei handelt es sich z.B. um Lebensmittel- oder Getränkereste, Farbstoffe, Etiketten, Klebstoffe, Reste von Papier, Metall, Glas, Kunststoffen. Bevorzugt ist ein Fremdstoffanteil in den Abfällen unter etwa 5 %, besonders bevorzugt unter 1 %. Kein Problem stellen Fremdstoffe dar, die unter den Bedingungen der zyklisierenden Depolymerisation (Vakuum, Temperatur) nicht flüchtig sind oder nichtflüchtige Zwischenprodukte oder flüchtige Zersetzungsprodukte mit einem Siedepunkt bei Atm.-Druck von < 150°C bilden. Nicht flüchtige Fremdstoffe oder Zersetzungsprodukte sammeln sich im Rückstand der zyklisierenden Depolymerisation. Flüchtige Zersetzungsprodukte unterhalb des angegebenen Siedepunkts können das gebildete Lactid nicht verunreinigen, wenn sie nicht in einem ähnlichen Temperaturbereich kondensieren wie Lactid. Fremdstoffe, die selbst mit Lactid reagieren oder die bei der Depolymerisation Zersetzungsprodukte bilden, die mit Lactid reagieren, sollen bevorzugt entfernt werden, bevor sie dem erfindungsgemäßen Verfahren unterworfen werden.

*Methoden zur Lactid- oder Glycolid-Reinigung:* Das erfindungsgemäße Verfahren benutzt bekannte Verfahren zur Reinigung von Rohlactid oder Rohglycolid aus der zyklisierenden Depolymerisation. Solche Verfahren sind z.B. die Rektifikation (EP 0 893 462 B1), Kristallisation aus der Schmelze (US 3,621,664 oder WO 2007/148) oder aus einem Lösungsmittel. Der Offenbarungsgehalt dieser Dokumente hinsichtlich der erwähnten Reinigungsmethoden wird durch Bezugnahme auch zum Gegenstand dieser Patentanmeldung gemacht.

Neben der Abtrennung von Resten von Wasser, Milchsäure, linearen und höheren zyklischen Oligomeren, ist die Trennung oder Einstellung des Gehalts an meso-Lactid, L- und D-Lactid im Reinlactid ein notwendiger Bestandteil der Lactidreinigung.

Die Qualität des PLAs, das aus Lactid hergestellt wird, das nach dem erfindungsgemäßen Verfahren erzeugt wird, hängt ab von dessen Gehalt an D-Lactid und meso-Lactid-Einheiten. Mit steigendem Gehalt an D-Milchsäure-Einheiten im vorwiegend aus L-Lactid-Einheiten aufgebauten PLA sinken dessen Schmelzepunkt, Wärmeformbeständigkeit, Kristallisationsgeschwindigkeit sowie Kristallisationsgrad. Wenn dieser Gehalt an D-Einheiten im PLA 6-8% übersteigt, ist das Polymer amorph, der Schmelzpunkt fällt mit der Glasübergangstemperatur von z.B. 55°C zusammen. Gleiches gilt für meso-Lactid und L-Lactid in einem vorwiegend aus D-Einheiten aufgebauten PLA.

Bei einem PLA-Recycling-Verfahren ist nicht vorherzusehen, mit welchem Gehalt an L- und D-Milchsäure-Einheiten die PLA-Abfälle ankommen. Um die Qualität des Endprodukts unabhängig von der Beschaffenheit der Abfälle zu machen, ist eine Abtrennung oder Konzentrationseinstellung der optischen Lactid-Isomere bevorzugt. Auf diese Weise kann die Lactidzusammensetzung im Rohstoff der Ringöffnungspolymerisation und damit im PLA nach Wunsch eingestellt werden.

meso-Lactid kann aus L-Lactid oder D-Lactid sowohl durch destillative Methoden als auch durch Kristallisation aus der Schmelze entfernt bzw. dessen Gehalt eingestellt werden. D- und L-Lactid können durch Kristallisation aus der Schmelze oder aus einer Lösung getrennt werden.

*PLA-Schmelze:* PLA hat je nach der Isomeren-Reinheit des Lactids, aus dem es aufgebaut ist, eine Schmelztemperatur zwischen 40°C und 170°C. PLA, das Stereokomplex-Kristallite enthält, hat eine Schmelztemperatur bis zu 230°C. Das erfindungsgemäße Verfahren benutzt partielle Hydrolyse und zyklisierende Depolymerisation in der schmelzflüssigen Phase, um das Verfahren auf bequeme und wirtschaftliche Weise kontinuierlich zu gestalten. Grundvoraussetzung für das Verfahren ist deshalb eine fließfähige Schmelze. Bei PLA-Abfällen muss aufgrund der heterogenen Zusammensetzung damit gerechnet werden, dass sie PLA mit sehr unterschiedlichen Schmelztemperaturen enthalten.

*Hydrolysierendes Medium:* Erfindungsgemäß sind darunter Wasser, Milchsäure, Glycolsäure oder ein Gemisch aus Wasser und Milchsäure sowie Mischungen aus Wasser und Glycolsäure zu verstehen. Reine Milchsäure bewirkt mit PLA zwar keine Hydrolyse, sondern eine Umesterung. Die Wirkung - der Abbau zu PLA-Oligomeren - ist jedoch dieselbe, wie bei der Hydrolyse. Eine Unterscheidung erübrigt sich deshalb für die Zwecke dieser Erfindung. Milchsäure enthält in industriell verfügbaren Qualitäten jedoch stets Wasser und fördert die Hydrolyse katalytisch. Der erfindungsgemäß verwendete Begriff "Milchsäure" schließt somit geringe Anteile von Wasser ein. Auch andere wasserhaltige Säuren fördern die Hydrolyse, müssen jedoch anschließend aus dem Produkt entfernt werden, da sie anders als Milchsäure die Depolymerisation zum Lactid oder dessen Polymerisation zu PLA stören.

*Partielle Hydrolyse:* Wie alle Polyester ist auch PLA bzw. PGA der hydrolytischen Spaltung der Polymerketten zugänglich. Was bei der Polymerisation und Verarbeitung vermieden werden muss, wird im erfindungsgemäßen Verfahren dazu benutzt, das Polymer gezielt bis auf eine gewünschte Molmasse abzubauen. Obwohl die Hydrolyse des PLA bereits bei Temperaturen unterhalb des Schmelzpunkts abläuft, sind die dafür benötigten Verweilzeiten oder die benötigte Wasserkonzentration im Polymer sehr hoch. Ein technischer Prozess ist dann vorteilhaft, wenn er in kurzen Verweilzeiten zum Ziel führt, was zu geringen Baugrößen bei den Apparaten führt. Der erfindungsgemäße Prozess benutzt darüber hinaus die geringste mögliche Wassermenge zur Hydrolyse, mit dem Ziel, nur genau die gewünschte Molmasse zu erreichen. Damit wird überschüssiges Wasser vermieden, das nach Abschluss der Hydrolysereaktion mit Aufwand an Energie und Apparaten wieder entfernt werden muss. Eine Hydrolyse mit deutlich größerer Wassermenge führt darüber hinaus zur stärkeren partiellen Racemisierung (JP 2009-249508; M. Faisal et al, Asian Journal of Chemistry Vol. 19, No. 3 (2007), S. 1714).

Die erwünschte mittlere Molmasse (Zahlenmittel) nach der Hydrolyse beträgt bei Lactid-Oligomeren zwischen 162 und 10.000 g/mol bzw. bei Glycolid-Oligomeren zwischen 134 und 10.000 g/mol, bevorzugt je zwischen 400 und 2000 g/mol. Dieser Molmassenbereich eignet sich besonders für die nachfolgende zyklisierende Depolymerisation zum Lactid bzw. Glycolid, wie aus Angaben zur technischen Gestaltung der Lactidherstellung oder Glycolidherstellung bekannt ist.

*Zyklisierende Depolymerisation:* Diese Reaktion ist die Umkehrreaktion der Ringöffnungspolymerisation, die in fast allen industriellen Prozessen zum Aufbau des PLA aus Lactid bzw. PGA aus Glycolid verwendet wird. Sie ist am fertigen PLA bzw. PGA unerwünscht, weil sie während der Verarbeitung zum Molmassenabbau und damit zur Verschlechterung der Produkteigenschaften führt. Bei der Herstellung von Lactid wird sie genutzt, um aus dem durch Polykondensation der Milchsäure erhaltenen Oligomer ein Rohlactid herzustellen, das nach Reinigung den Ausgangsstoff für die Ringöffnungspolymerisation bildet.

Der in der ersten Stufe durchgeführte partielle hydrolytische Abbau zu den jeweiligen Oligomeren ausgehend von den polymeren Materialien PLA bzw. PGA zu dem angegebenen mittleren Molekulargewicht erfolgt dabei durch entsprechende Wahl der jeweiligen Parameter, wie z.B. der Menge des zu hydrolysierenden Mediums bezüglich den polymeren Materialien sowie Temperatur und Druck. Die entsprechenden Parameter können anhand einfacher Experimente eingestellt und ermittelt werden.

Eine bevorzugte Ausführungsform sieht vor, dass das hydrolysierende Medium ausgewählt ist aus der Gruppe bestehend aus Wasser, Milchsäure, Glycolsäure, Mischungen aus Wasser und Milchsäure sowie Mischungen aus Wasser und Glycolsäure. Sofern reine Milchsäure bzw. reine Glycolsäure eingesetzt werden, wird erfindungsgemäß hierunter verstanden, dass diese reinen Substanzen zumindest noch Spuren von Wasser enthalten.

In einer weiteren vorteilhaften Ausführungsform beträgt das zahlengemittelte Molekulargewicht der in Stufe a) erhaltenen PLA- bzw. PGA-Oligomere zwischen 400 und 2.000 g/mol. Das zahlengemittelte Molekulargewicht wird dabei durch Säure-Base-Titration der Carboxylgruppen der jeweiligen Oligomere bestimmt.

Weiter ist vorteilhaft, wenn 50 mmol bis 10 mol, bevorzugt 100 mmol bis 5 mol, insbesondere 0,5 mol bis 3 mol an hydrolysierendem Medium pro 1 kg der Masse an PLA bzw. PGA zugegeben wird.

Bevorzugte Parameter, die während des hydrolytischen Abbaus in Stufe a) eingestellt werden, sind dabei:
α) eine Temperatur von 130 bis 300 °C, bevorzugt von 150 bis 250 °C, besonders bevorzugt von 190 bis 230 °C,
β) ein Druck von 5 bis 500 bar, bevorzugt von 10 bis 300 bar, besonders bevorzugt von 20 bis 200 bar, und/oder
γ) eine Verweilzeit von 0,1 bis 50 min, bevorzugt von 1 bis 15 min, besonders bevorzugt von 1 bis 5 min.

Vor der zyklisierenden Depolymerisation in Stufe b) den Oligomeren ein Katalysator zugegeben wird, insbesondere in einer Konzentration von 0,01 bis 50 mmol/kg Oligomere, weiter bevorzugt von 0,1 bis 10 mmol/ kg Oligomere, zugegeben wird.

Bevorzugte polymere Ausgangsmaterialien stammen dabei teilweise oder ganz aus Abfallmaterial, das beispielsweise als Ausschussmaterial bei der Polymerisation von Lactid bzw. Glycolid anfällt. Bevorzugt werden dabei die PLA- bzw. PGA-Abfälle vor Aufgabe in Stufe a)
α) durch Sortieren, Waschen und andere Trennmethoden von Fremdstoffen gereinigt, so dass bevorzugt der Anteil an Fremdstoffen und/oder Verunreinigungen weniger als 5 Gew.-%, weiter bevorzugt weniger als 1 Gew.-%, bezogen auf die Masse an PLA bzw. PGA beträgt, und/oder
β) zerkleinert, so dass die maximale Abmessung der erhaltenen zerkleinerten Abfälle 15 mm beträgt.

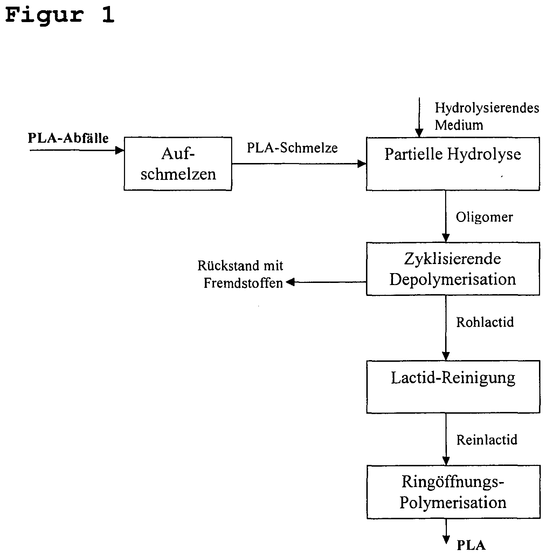

Weiter ist es vorteilhaft, wenn die in Stufe a) erhaltenen Oligomeren
α) zu Milchsäure bzw. Glycolsäure, die in eine Polykondensationsstufe aufgegeben wird, wobei aus Milchsäure bzw. Glycolsäure deren jeweilige Oligomere hergestellt werden,
β) in eine Polykondensationsstufe von Milchsäure bzw. Glycolsäure, wobei aus Milchsäure bzw. Glycolsäure deren jeweilige Oligomere hergestellt werden, und/oder
γ) zu den aus einer Polykondensationsstufe von Milchsäure bzw. Glycolsäure erhaltenen Oligomeren,
zugegeben werden und das so erhaltene Oligomerengemisch der zyklisierenden Depolymerisation (Stufe b)) zugeführt wird. Diese Ausführungsform ist insbesondere in Figur 1 dargestellt.

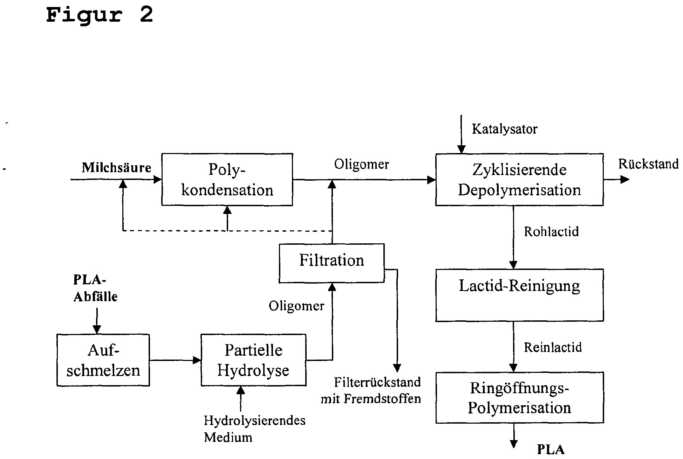

Die zuvor beschriebene bevorzugte Ausführungsform sieht somit vor, dass die zunächst durch hydrolytischen Abbau erzeugten Oligomeren für weitere Prozessschritte bei der Herstellung von Oligomeren von Milchsäure bzw. Glycolsäure verwendet werden. Dies kann beispielsweise durch Zugabe der in Stufe a) (siehe hierzu beispielsweise Figur 2 und zugehörige Ausführungen) erhaltenen Oligomeren zu Milchsäure bzw. Glycolsäure, d.h. vor Oligomerisierung dieser Monomeren, oder auch direkt in eine derartige Kondensationsstufe zu den jeweiligen Oligomeren erfolgen. Ebenso können die aus Stufe a) erhaltenen Oligomeren auch mit aus weiteren Prozesslinien stammenden Oligomeren von Milchsäure bzw. Glycolsäure vereinigt werden.

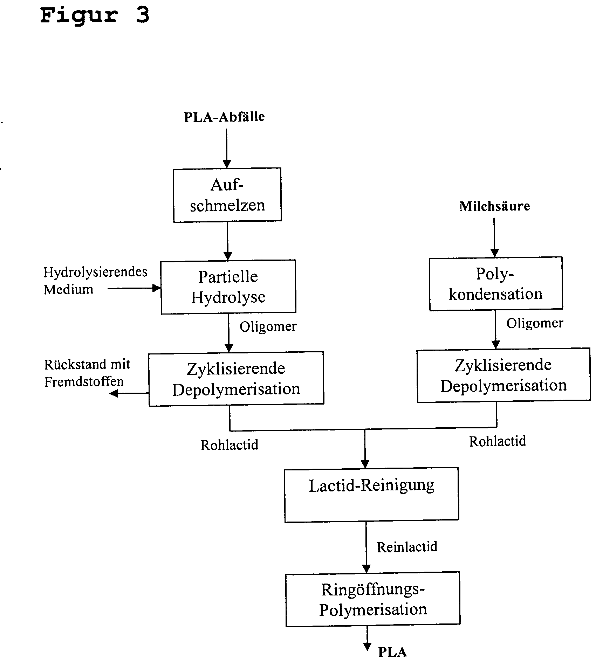

Ebenso kann vorgesehen sein, dass das durch die zyklisierende Depolymerisation (Stufe b)) erhaltene Lactid bzw. Glycolid mit Lactid bzw. Glycolid, das durch Polykondensation von Milchsäure bzw. Glycolsäure zu Oligomeren und zyklisierender Depolymerisation dieser Oligomere erhalten wurde, vermischt wird (siehe hierzu beispielsweise Figur 3 und zugehörige Ausführungen).

Zudem kann es vorteilhaft sein, das aus Stufe b) erhaltene Lactid in verschiedene Fraktionen zu zerlegen. Die hierbei erhaltenen Lactid-Fraktionen können dabei an meso-Lactid angereichert und/oder abgereichert werden bzw. an L-Lactid, D-Lactid und/oder meso-Lactid an- oder abgereichert werden. Der Gehalt der jeweiligen Lactid-Fraktion wird dabei mit dem Lactid-Gehalt der unmittelbar aus Stufe b) erhaltenen Lactid-Fraktion verglichen. Die Auftrennung der jeweiligen Fraktionen kann dabei beispielsweise durch fraktionierende Destillation oder durch die eingangs beschriebenen Trenn- und Reinigungsverfahren erfolgen.

Insbesondere wird das nach Stufe b) erhaltene Lactid dabei in eine - verglichen mit dem unmittelbar aus Stufe b) erhaltenen Lactid -
α) an meso-Lactid abgereicherte Fraktion und eine an meso-Lactid angereicherte Fraktion aufgetrennt, oder
β) in eine an L-Lactid angereicherte Fraktion, eine an D-Lactid angereicherte Fraktion und/oder eine an meso-Lactid angereicherte Fraktion aufgetrennt, wobei bevorzugt die Konzentration dieser Komponenten in der Fraktion ≥ 50 Gew.-%, bevorzugt ≥ 90 Gew.-%, besonders bevorzugt ≥ 98 Gew.-% beträgt.

Weiter ist es bevorzugt, wenn
α) die Oligomeren vor Schritt b) und/oder
β) das erhaltene Lactid bzw. Glycolid nach Schritt b) einer Reinigung, insbesondere einer destillativen Reinigung und/oder einer Umkristallisation unterzogen wird/werden.

Gemäß einer zudem bevorzugten Ausführungsform wird das PLA bzw. PGA in die Stufe a) in aufgeschmolzenem Zustand aufgegeben oder während Stufe a) aufgeschmolzen. Die Kontaktierung des PLA bzw. PGA in Stufe a) mit dem hydrolysierenden Medium kann somit also bereits in geschmolzenem Zustand erfolgen; ebenso ist es auch möglich, dass die Kontaktierung vor Aufschmelzen des PGA bzw. PLA erfolgt. Die partielle Hydrolyse wird dabei bevorzugt in geschmolzenem Zustand durchgeführt.

Insbesondere eignet sich das Verfahren zur kontinuierlichen Verfahrensführung.

Erfindungsgemäß wird ebenso ein Verfahren zur Herstellung von PLA bzw. PGA angegeben, wobei von Lactid bzw. Glycolid ausgegangen wird und diese Monomere in einer Ringöffnungspolymerisation zu PLA bzw. PGA umgesetzt werden. Kennzeichnend und erfindungsgemäß dabei ist, dass zumindest ein Teil oder die Gesamtheit des eingesetzten Lactids bzw. Glycolids nach einem zuvor beschriebenen Rückgewinnungsverfahren hergestellt wurde.

Die Erfindung betrifft weiterhin eine Vorrichtung zur kontinuierlichen Rückgewinnung von Lactid aus PLA oder Glycolid aus PGA, umfassend
a) eine Vorrichtung (1) zum Aufschmelzen von PLA bzw. PGA und/oder eine Vorrichtung zur Zuführung einer PLA- bzw. PGA-Schmelze, wobei die Vorrichtung (1) zum Aufschmelzen ein Extruder oder ein Schmelzrost ist,
b) eine stromabwärts der Vorrichtung (1) zum Aufschmelzen und/oder der Vorrichtung zur Zuführung einer PLA- bzw. PGA-Schmelze angeordnete Hydrolysevorrichtung(2) zur Durchführung einer partiellen Hydrolyse der PLA- bzw. PGA-Schmelzen zu PLA- bzw. PGA-Oligomeren, wobei die Hydrolysevorrichtung (2) einebeheizbare Rohrstrecke aufweist, sowie
c) einen stromabwärts der Hydrolysevorrichtung (2) angeordneten Depolymerisationsreaktor (9), der als Umlaufverdampfer, Fallfilmverdampfer, Dünnschichtverdampfer oder als eine Kombination aus zwei oder drei dieser Bauarten ausgeführt ist.

Zentraler Bestandteil der erfindungsgemäßen Vorrichtung ist somit eine Hydrolysevorrichtung, in der die partielle Hydrolyse des PLAs bzw. PGAs durchgeführt wird.

Der Hydrolysevorrichtung vorgeschaltet ist dabei eine Vorrichtung zum Aufschmelzen von PLA bzw. PGA bzw. eine Vorrichtung zur Zuführung einer PLA- oder PGA-Schmelze in die Hydrolysevorrichtung.

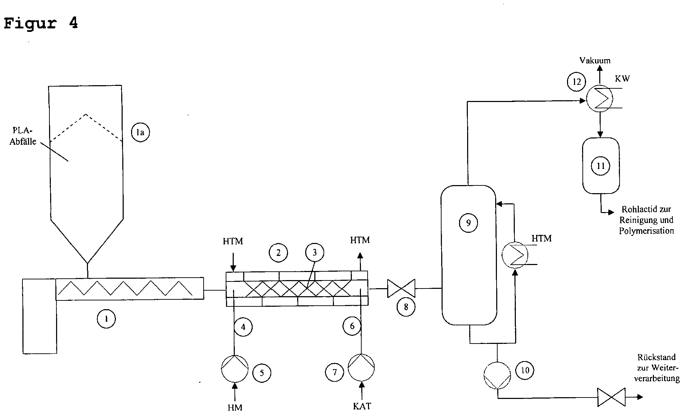

Die erfindungsgemäße Vorrichtung umfasst somit stromabwärts der Vorrichtung zum Aufschmelzen und/oder der Vorrichtung zur Zuführung einer PLA- bzw. PGA-Schmelze eine Hydrolysevorrichtung zur Durchführung einer partiellen Hydrolyse der PLA- bzw. PGA-Schmelzen zu PLA- bzw. PGA-Oligomeren, die mit der Vorrichtung zum Aufschmelzen und/oder der Vorrichtung zur Zuführung einer PLA- bzw. PGA-Schmelze über eine Schmelzeleitung für die Schmelze in Verbindung steht, wobei die Hydrolysevorrichtung dem Depolymerisationsreaktor vorgeschaltet ist (siehe hierzu Figur 4).

Erfindungsgemäß werden somit zunächst PLA bzw. PGA in einer Schmelzvorrichtung aufgeschmolzen und diese Schmelze der Hydrolysevorrichtung zugeführt bzw. bereits eine Schmelze der Hydrolysevorrichtung zugeführt, wobei in der Hydrolysevorrichtung die partielle Hydrolyse des PLAs bzw. PGAs stattfindet. Die hierbei entstehenden PLA- bzw. PGA-Oligomeren werden danach dem Depolymerisationsreaktor zugeführt, in dem eine zyklisierende Depolymerisation zu Lactid bzw. Glycolid durchgeführt wird.

Bevorzugt ist dabei, wenn die Vorrichtung zur Zuführung einer PLA- bzw. PGA-Schmelze eine Schmelzeleitung und/oder Schmelzepumpe ist, und/oder die beheizbare Rohrstrecke der Hydrolysevorrichtung (2) beheizbare oder unbeheizbare statische Mischelemente aufweist.

Weiter vorteilhaft verfügt die Hydrolysevorrichtung über
a) eine Eintragsmöglichkeit für ein hydrolysierendes Medium, die bevorzugt in die Schmelzezuführung der Hydrolysevorrichtung mündet, und/oder
b) eine Eintragsmöglichkeit für einen Depolymerisationskatalysator, die bevorzugt in die Schmelzeabführung der Hydrolysevorrichtung mündet.

Ebenso ist es bevorzugt, wenn der Depolymerisationsreaktor eine Abzugsvorrichtung aufweist, über die nicht umgesetzte Schmelze ausgetragen werden kann. Der Depolymerisationsreaktor kann einen kopfseitig angeordneten Abzug für Lactid- bzw. Glycoliddämpfe aufweisen, der in eine Kondensationsvorrichtung für Lactid- bzw. Glycoliddämpfe mündet.

Eine weitere vorteilhafte Ausführungsform sieht vor, dass die Kondensationsvorrichtung eine Bevorratungsvorrichtung für das erzeugte Lactid bzw. Glycolid und/oder eine Reinigungsvorrichtung für Lactid bzw. Glycolid nachgeschaltet ist.

Zwischen der Hydrolysevorrichtung und dem Depolymerisationsreaktor kann ein Polykondensationsreaktor zur Herstellung von PLA- bzw. PGA-Oligomeren durch Polykondensation von Milchsäure bzw. Glycolsäure angeordnet sein, wobei der Auslass der Hydrolysevorrichtung vor, in oder nach dem Polykondensationsreaktor mündet.

Der Auslass des Depolymerisationsreaktors kann mit dem Auslass eines Reaktors, der in einer ersten Stufe mittels Polykondensation von Milchsäure bzw. Glycolsäure PLA- bzw. PGA-Oligomeren und in einer zweiten Stufe aus diesen PLA- bzw. PGA-Oligomeren mittels zyklisierender Depolymerisation Lactid bzw. Glycolid erzeugt, zusammenlaufen.

Weiter umfasst die vorliegende Erfindung eine Vorrichtung zur Herstellung von PLA bzw. PGA durch Ringöffnungspolymerisation von Lactid bzw. Glycolid, die eine vorangehend beschriebene Vorrichtung zur kontinuierlichen Rückgewinnung von Lactid aus PLA oder Glycolid aus PGA, eine Vorrichtung zur Reinigung des Lactids, sowie eine nachgeschaltete Ringöffnungspolymerisationsvorrichtung enthält.

Die vorliegende Erfindung wird anhand der nachfolgenden Ausführungsformen sowie der beigefügten Figuren näher beschrieben, ohne die Erfindung auf die dort dargestellten speziellen Ausführungsformen zu beschränken.

Dabei zeigt:
- Figur 1: den generellen Ablauf eines Verfahrens gemäß der vorliegenden Erfindung ausgehend von PLA-Abfällen bis hin zur Herstellung von frischem PLA durch Ringöffnungspolymerisation.
- Figur 2: ein Verfahren zur Herstellung von PLA mit Einspeisung von partiell hydrolysierten PLA-Abfällen.
- Figur 3: ein Verfahren zur Herstellung von PLA mit Einspeisung von aus PLA-Abfällen hergestelltem Rohlactid sowie
- Figur 4: eine schematische Darstellung einer erfindungsgemäßen Vorrichtung zur Durchführung des Verfahrens.

Figur 1 zeigt das prinzipielle Schema des Ablaufs eines erfindungsgemäßen Verfahrens anhand von PLA-Abfällen als beispielhaftes Ausgangsmaterial. In einem ersten Schritt erfolgt ein Aufschmelzen der PLA-Abfälle; die so erzeugte PLA-Schmelze wird mit einem hydrolysierenden Medium versetzt und anschließend hydrolysiert, wodurch ein teilweiser Abbau der PLA-Polymerkette erfolgt und somit PLA-Oligomere erzeugt werden. Nach dieser partiellen Hydrolyse erfolgt eine sich anschließende zyklisierende Depolymerisation; hierbei wird ein Rohlactid erhalten. Als Nebenprodukt fällt dabei unter Umständen ein Rückstand mit Fremdstoffen an. Das so erzeugte Rohlactid kann einer optionalen Lactid-Aufreinigung, beispielsweise einer destillativen Reinigung oder einer Reinigung durch Umkristallisation unterzogen werden. Im in Figur 1 dargestellten Beispiel wird das so erzeugte Reinlactid gleich im Anschluss einer Ringöffnungspolymerisation unterzogen, wodurch neues Polylactid hergestellt werden kann.

Das erfindungsgemäße Verfahren kann in einer eigenen Recyclinganlage (Figur 1) ausgeführt werden, die aus Vorrichtungen für Aufschmelzen, Hydrolyse, zyklisierende Depolymerisation, die Lactidreinigung und die Ringöffnungspolymerisation besteht. Insgesamt muss für dieses Verfahren also eine vollständige PLA-Anlage zur Verfügung stehen, die denselben Umfang hat, gemessen als Zahl der Prozessschritte, wie eine PLA-Anlage, die von Milchsäure ausgeht.

Das Reinlactid aus einer solchen Recyclinganlage kann auch als Rohstoff für die Ringöffnungspolymerisation zu einer Produktionsanlage für jungfräuliches PLA transportiert und dort für die PLA-Produktion verwendet werden. Auf diese Weise kann auf die Vorrichtung für die Ringöffnungspolymerisation verzichtet werden.

Figur 2 beschreibt ein Verfahren zur Herstellung von PLA mit Einspeisung von partiell hydrolysierten PLA-Abfällen, die gemäß dem Verfahren der vorliegenden Erfindung erzeugt wurden. Der in Figur 2 im linken unteren Zweig dargestellte Schritt der Aufarbeitung der PLA-Abfälle entspricht im Wesentlichen der bereits in Figur 1 erläuterten Verfahrensführung. Dabei werden PLA-Abfälle in einer ersten Stufe aufgeschmolzen und einer partiellen Hydrolyse unter Zusatz eines hydrolysierenden Mediums zugeführt. Die so erzeugten Oligomere können beispielsweise durch Filtration von Fremdstoffen befreit werden. Die gereinigten Oligomere werden mit den Oligomeren, die von der Polykondensation von Milchsäure herrühren, vereinigt. Alternativ und/oder zusätzlich hierzu können die Oligomere aus der partiellen Hydrolyse der PLA-Abfälle jedoch auch bereits der als Edukt eingesetzten Milchsäure zugesetzt und in die Polykondensationsstufe aufgegeben werden oder direkt in die Polykondensationsstufe der Milchsäure aufgegeben werden. Diese Möglichkeiten sind in Fig. 2 durch die gestrichelten Pfeile angedeutet. Die so erhaltenen Oligomere werden der zyklisierenden Depolymerisation zugeführt, wobei ein Rohlactid entsteht. Die zyklisierende Depolymerisation wird bevorzugt unter Zugabe eines Katalysators durchgeführt, verbleibende Rückstände werden aus dieser Stufe entfernt. Nach optionaler Aufreinigung, beispielsweise durch Destillation oder Kristallisation, kann das Reinlactid erneut einer Ringöffnungspolymerisation zugeführt und zu PLA umgewandelt werden. Ebenso ist jedoch auch eine weitere Reinigung des Reinlactids durch Abtrennung von optischen Isomeren denkbar.

Es ist ebenso vorteilhaft, das erfindungsgemäße Verfahren als Teil eines industriellen PLA-Verfahrens und eine erfindungsgemäße Anlage als Teil einer PLA-Produktionsanlage zu betreiben. In diesem Fall muss die PLA-Produktionsanlage lediglich um eine Seitenlinie mit Vorrichtungen zum Aufschmelzen und zur partiellen Hydrolyse der PLA-Abfälle erweitert werden (Figur 2). Nach Einspeisen des partiellen Hydrolysats vor, in oder nach der Polykondensation ersetzt es einen Teil des Rohstoffs Milchsäure. Es werden für das rezyklierte Material also keine weiteren Prozessstufen bis zum PLA benötigt, sondern die vorhandenen mitbenutzt. Es leuchtet unmittelbar ein, dass diese Möglichkeit wirtschaftliche Vorteile bietet.

Figur 3 zeigt eine weitere Variante der Verfahrensführung zur Herstellung von PLA, die auf das erfindungsgemäße Verfahren zur Rückgewinnung von Lactid aus PLA-Abfällen zurückgreift. In zwei parallelen Zweigen der Verfahrensführung wird einerseits Rohlactid durch partielle Hydrolyse und zyklisierende Depolymerisation von PLA-Abfällen erzeugt (siehe Figur 3, linker Zweig); diese Verfahrensführung verläuft identisch mit den ersten drei Schritten der in Figur 1 dargestellten Verfahrensführung. In einem zweiten Zweig (siehe Figur 3, rechter Zweig) erfolgt ebenso die Herstellung von Rohlactid, allerdings aus Milchsäure, wobei eine Polykondensation mit anschließender zyklisierender Depolymerisation erfolgt. Beide Rohlactidströme können vereinigt einer Aufreinigung unterzogen werden, wobei Reinlactid erhalten wird. Dieses Reinlactid kann in direktem Anschluss einer Polymerisation zu PLA zugeführt werden.

Diese Ausführungsform der Erfindung ist z.B. dann von Vorteil, wenn während der zyklisierenden Depolymeri-sation größere Mengen von Fremdstoffen im nicht umgesetzten Rückstand zurückbleiben oder in die Lactid-Dämpfe übergehen, die den PLA-Prozess in der Hauptlinie stören würden. In dieser Variante wird das Rohlactid aus den PLA-Abfällen vor der Lactidreinigung der Hauptlinie zugegeben, so dass die Vorrichtungen der Lactidreinigung und der folgenden ringöffnenden Polymerisation der Hauptlinie für das rezyklierte Material mitbenutzt wird. Auch in dieser Variante ergeben sich noch erhebliche Einsparungen an Apparaten. Der Vollständigkeit halber sei erwähnt, dass das Rohlactid aus den PLA-Abfällen auch vor, in oder nach dem Polykondensationsschritt der Hauptlinie zugesetzt werden kann.

Figur 4 zeigt eine Vorrichtung zur Durchführung des erfindungsgemäßen Verfahrens anhand einer beispielhaften Ausgestaltungsform. Hierin bedeuten:
HM: hydrolysierendes Medium (Wasser, Milchsäure, Gemisch aus Wasser und Milchsäure, etc.)
KAT: Katalysator
HTM: Wärmeträgermedium

Die Vorrichtung gemäß Figur 4 umfasst einen Einschneckenextruder 1 zum Aufschmelzen der zerkleinerten, vorsortierten und vorgereinigten Abfälle, die einem Vorlagebehälter entnommen werden. Mit dem Extruderausgang ist eine von außen beheizbare, druckfeste Rohrstrecke 2 verbunden, die innen mit statischen Mischelementen 3 ausgerüstet ist. Am Eingang der Rohrstrecke dient z.B. eine Kapillare 4 aus Edelstahl der Zufuhr von Wasser zur partiellen Hydrolyse. Die Kapillare ist mit einer Dosierpumpe 5 versehen, die gegen den Schmelzedruck demineralisiertes Wasser oder verdünnte Milchsäure in die PLA-Schmelze dosiert. Eine zweite Kapillare 6 mit Dosierpumpe 7 ist am Ende der Rohrstrecke angeordnet. Sie dient zur Dosierung des flüssigen oder gelösten Katalysators für die Depolymerisation.

Am Ende der Rohrstrecke ist ein Druckhalteventil 8 angeordnet, durch das die Schmelze in den folgenden Depolymerisationsreaktor entspannt wird, der unter Vakuum steht.. Dieser Reaktor ist in diesem Beispiel als Umlaufverdampfer 9 ausgeführt. Diese Stufe ist mit einer Abzugsvorrichtung 10 ausgestattet, durch die die nicht umgesetzte Schmelze ausgetragen wird.

Die durch die Depolymerisationsreaktion entstandenen Lactid-Dämpfe werden aus der Reaktionsstufe abgezogen und in einer Kondensationsvorrichtung 12 verflüssigt. Das flüssige Rohlactid geht in einen Sammeltank 11 und von dort in die Lactid-Reinigung. Dort wird es zu polymerisierbarem Reinlactid umgewandelt.

Bei der hier beschriebenen Vorrichtung handelt es sich um ein Ausführungsbeispiel zur Illustration des Verfahrens. Die Beschreibung ist deshalb in keiner Weise als Einschränkung des Verfahrens zu verstehen. Das der Erfindung zugrunde liegende Verfahren kann auch in vielfältig abgewandelten Vorrichtungen durchgeführt werden, wie in der Verfahrensbeschreibung erwähnt.

Anhand der nachfolgenden Ausführungen werden bevorzugte Varianten der erfindungsgemäßen Verfahrensführung verdeutlicht.

PLA-Abfälle werden nach Sortierung und geeigneter mechanischer Vorreinigung aufgeschmolzen. Der Schmelze wird eine genau dosierte Menge an Wasser, Milchsäure oder einem Gemisch der beiden zugesetzt, die eine Hydrolyse zu einem Oligomer bewirkt. Das Oligomer wird einer zyklisierenden Depolymerisation unterworfen, die als Produkt ein Rohlactid erzeugt. Nach Reinigung dieses Lactids mit bekannten Verfahren kann es zu PLA ohne Einbuße von Eigenschaften polymerisiert werden. Das Verfahren kann besonders vorteilhaft in Verbindung mit einer Vorrichtung eingesetzt werden, die mit einer Anlage zur Herstellung von PLA-Neuware verbunden ist.

Die technische Gestaltung des erfindungsgemäßen Verfahrens erfolgt z.B. und bevorzugt so, dass die Abfälle mechanisch nach bekannten Verfahren durch Sortieren, Waschen und Separieren von Fremdstoffen gereinigt werden, so dass der Anteil an Fremdstoffen weniger als 5 Massen-%, bevorzugt weniger als 1 Massen-%, beträgt. Nach Zerkleinerung auf eine maximale Abmessung von 15 mm, die je nach Art und Eigenschaften der Abfälle vor oder nach dem Abtrennen von Fremdstoffen erfolgen kann, werden die Abfälle kontinuierlich einem Extruder zugeführt und aufgeschmolzen.

Der Extruder kann als Ein-, Zwei- oder Mehrwellenmaschine ausgeführt sein. Bevorzugt ist ein Einwellenextruder, der vollständig mit Schmelze gefüllt ist und einen Schmelzedruck aufbauen kann. Eine Entgasung zur Abtrennung von Feuchtigkeit ist während des Aufschmelzens nicht nötig, da die nachfolgende Hydrolyse ohnehin Wasser benötigt. Eine Maschine mit Entgasung bietet dann Vorteile, wenn Gase oder andere flüchtige Stoffe als Wasser beim Aufschmelzen entstehen, die den folgenden Prozess stören können. Anstelle eines Extruders eignet sich auch ein Schmelzrost, der ebenfalls die Möglichkeit der Entgasung bietet. Der Schmelzrost ist eine kostengünstige Alternative, die jedoch nur bei sehr geringen Fremdstoffanteilen infrage kommt, wenn keine Gefahr der Separation von Verunreinigungen aus der Schmelze droht, die den Rost verstopfen könnten.

An den Extruder schließt sich eine Hydrolysevorrichtung an, die im Wesentlichen aus einer beheizten Rohrstrecke besteht, welche der Schmelze die für die Hydrolysereaktion benötigte Verweilzeit zur Verfügung stellt. Um die Schmelze während der Hydrolyse weiter zu mischen und zu homogenisieren, kann die Rohrstrecke mit einem statischen Mischer ausgerüstet sein. Hierfür eignen sich z.B. Mischelemente vom Typ SMX der Fa. Sulzer. Der Durchmesser der Rohrstrecke nimmt bei größeren Durchsätzen zu. Ab einem Durchmesser von ca. 15 mm enthält die Rohrstrecke zur besseren Wärmezufuhr neben den statischen Mischelementen Rohre zum Hindurchleiten eines flüssigen Wärmeträgers. Diese Rohre können auch in der Form von statischen Mischelementen gewendelt oder aufgefaltet sein (z.B. Sulzer SMR) und benötigen dann keine zusätzlichen statischen Mischelemente.

Zwischen Extruder und Schmelzeleitung kann eine Schmelzepumpe, z.B. eine Zahnradpumpe, angeordnet werden. Dies ist erforderlich, wenn der Extruder den nötigen Vordruck für die Hydrolysevorrichtung nicht aufbringen kann. Im Falle, dass ein Schmelzrost zur Anwendung kommt, ist eine Schmelzepumpe zur Druckerhöhung obligatorisch.

Für die Hydrolyse eignen sich Wasser, Milchsäure oder Gemische aus beiden. Während Wasser die Auslegung der Hydrolysevorrichtung auf einen Druck von mindestens 30 bar erfordert, um bei der Reaktionstemperatur flüssig zu bleiben und Verdampfung zu verhindern, benötigt man bei Verwendung von Milchsäure korrosionsbeständige Materialien an der Einspeisestelle und an den stromabwärts liegenden, schmelzeberührten Teilen. Es ist vorteilhaft, Wasser, Milchsäure oder ihre Gemische vor der Einspeisung in die PLA-Schmelze auf die Schmelzetemperatur vorzuheizen, um Einfrieren der Schmelze an der Einspeisestelle zu verhindern.

Die für die Hydrolyse benötigte Wasser- oder Milchsäuremenge wird unter Druck wahlweise in den Extruder, zwischen Extruder und Hydrolysevorrichtung oder zwischen Schmelzepumpe und Hydrolysevorrichtung zudosiert. Vorzugsweise wird die mittlere Molmasse nach Ablauf der partiellen Hydrolyse über die Menge des zugegebenen hydrolysierenden Mediums eingestellt. In diesem Fall befindet sich die Schmelze nach der Hydrolysereaktion im Reaktionsgleichgewicht, wo auch die mittlere Molmasse im chemischen Gleichgewicht vorliegt. Ein "Überschießen" der Reaktion mit der Folge einer niedrigeren als der gewünschten mittleren Molmasse ist auf diese Weise ausgeschlossen. Bei Wasser als Hydrolysemedium liegt die zugegebene Menge bei bis zu 100 g H₂O/kg PLA, bevorzugt bei etwa 20 g H₂O/kg PLA. Der Druck beträgt ≥ 30 bar, nach oben begrenzt nur durch die Druckfestigkeit der Bauteile, in der Regel ca. 200 bar, und damit durch die Kosten des Apparats.

Es ist auch möglich, die gewünschte Molmasse durch Begrenzen der Verweilzeit und der Schmelzetemperatur während der Hydrolysereaktion einzustellen. In diesem Fall sind aber größere Schwankungen der Molmasse im Hydrolyseprodukt zu erwarten.

In einer Variante der Erfindung wird das hydrolysierende Medium in den Aufschmelzextruder eingespeist. Vorzugsweise wählt man in dieser Variante eine Schmelzetemperatur im oberen Bereich der angegebenen Spanne, um die für die Hydrolyse benötigte Verweilzeit kurz zu halten und die Hydrolysereaktion bereits im Extruder bis zur gewünschten Molmasse zu führen. In diesem Fall wird keine separate Hydrolysevorrichtung benötigt und die Schmelze kann direkt entspannt und dem Depolymerisationsreaktor zugeführt werden.

Nach der Hydrolysereaktion kann eine Filtration der Schmelze von Vorteil sein, die Verunreinigungen und Fremdstoffe zurückhält, die als Partikel mit Abmessungen über etwa 50 Mikrometern vorliegen (z.B. verkohlte Partikel). Dies ist u.a. dann von Nutzen, wenn dort mit Lactid reaktionsfähige Fremdstoffe oder deren Zersetzungsprodukte entfernt werden können.

Die Schmelzetemperatur am Ende des Aufschmelzvorgangs wird so gewählt, dass alle PLA-Partikel aufgeschmolzen sind und für die Hydrolyse keine weitere Temperaturerhöhung nötig ist, so dass sie innerhalb einer Verweilzeit zwischen 1 und 30 Minuten vollständig abgelaufen ist, bevorzugt zwischen 1 bis 5 Minuten. Die Schmelzetemperatur während der Hydrolysereaktion liegt zwischen 150°C und 250°C, bevorzugt zwischen 190°C und 230°C.

Nach der Hydrolyse liegt ein bei der Schmelzetemperatur dünnflüssiges Oligomer der Milchsäure vor. Es unterscheidet sich in der Molmasse nicht von dem Oligomer, das durch Polykondensation der Milchsäure im ersten Schritt der Lactidherstellung bei der Ringöffnungspolymerisation entsteht. Unterschiede sind der Gehalt an Polymerisationskatalysator und Stabilisator sowie gegebenenfalls an Fremdstoffen und deren Zersetzungsprodukten aus den PLA-Abfällen. Dieses Milchsäureoligomer wird im zweiten Schritt des erfindungsgemäßen Verfahrens in Lactid umgesetzt. Diese Reaktion ist die Umkehrreaktion der Ringöffnungspolymerisation, die in fast allen industriellen Prozessen zum Aufbau des PLA aus Lactid verwendet wird. Deshalb sind auch dieselben Katalysatoren - sowohl in der zyklisierenden Depolymerisation als auch in der ringöffnenden Polymerisation - aktiv. Gewöhnlich enthält PLA und damit PLA-Abfall Zinnverbindungen als Polymerisationskatalysator. Je nach Herkunft und Qualität der PLA-Abfälle wird deshalb Katalysator für die zyklisierende Depolymerisation zwischen 0 und 20 mmol/kg zugegeben, in den meisten Fällen genügen jedoch 0 bis 10 mmol/kg. Als Katalysator eignen sich bevorzugt organische Zinnverbindungen, z.B. Zinn(II)-oktoat. Es eignen sich jedoch auch alle anderen für die Ringöffnungspolymerisation der Lactide bekannten Katalysatoren.

Das erfindungsgemäße Verfahren wird anhand der nachfolgenden Beispiele näher erläutert. Hierzu wurden folgende analytische Methoden verwendet:

### Carboxylendgruppen des PLA-Hydrolysats, Molmasse der PLA-Oligomeren:

Das PLA-Oligomer wird in Aceton gelöst. Nach Zugabe von Methanol wird die Lösung mit 0,1 N benzylalkoholischer KOH-Lösung titriert. Der Endpunkt wird potentiometrisch erfasst. Aus der Carboxylendgruppenkonzentration ("COOH"), gemessen in mmol/kg, kann das Zahlenmittel der Molmasse berechnet werden nach der Gleichung Mn = 10⁶/COOH.

### Carboxylgruppen im Lactid:

Die Lactidprobe wird in Methanol gelöst und anschließend auf die gleiche Weise titriert wie bei der Carboxylendgruppenbestimmung im PLA-Oligomer.

### Bestimmung der Intrinsischen Lösungsviskosität:

Die abgewogene Polymermenge wird in einem definierten Volumen Chloroform gelöst. In einem Ubbelohde-Kapillarviskosimeter, das sich in einem auf 30°C +/- 0,1°C eingestellten thermostatisierten Wasserbad befindet, wird die Durchlaufzeit der Lösung und des reinen Lösungsmittels gemessen. Der Quotient aus beiden ist die relative Lösungsviskosität. Sie wird mit der Einpunktmethode nach J. Dorgan et al., J. Polym. Sci.: Part B: Polym. Physics, Vol. 43, S. 3100-3111, (2005), in die intrinsische Viskosität (I.V.) umgerechnet. Die I.V. steht mit dem Gewichtsmittel M_{w} der Molmasse des Polymers in Verbindung, die mit der sog. Mark-Houwink-Gleichung beschrieben wird. Für das Stoffpaar PLA/Chloroform lautet die Gleichung (J. Dorgan, a.a.O.):
I.V. = K * Mw * a , mit K = 1,53 * 10⁻⁹ dl/g,
a = 0,759

### Optische Isomere des Lactids:

Die Lactid-Probe wird in einem Gemisch aus 90/10 ml/ml n-Hexan/Ethanol gelöst. Die gelösten Komponenten werden mit HPLC auf einer chiralen Säule getrennt und mit einem UV-Detektor bei 223 nm analysiert.

### D-Anteil in Milchsäure und PLA:

Eine Probe aus PLA oder einem PLA-Oligomer wird mit 1-N Natronlauge am Rückfluss siedend hydrolysiert und nach Abkühlen neutralisiert. Die neutralisierte Probe wird mit 3 millimolarer Kupfersulfatlösung im Verhältnis 1/9 ml/ml vermischt und mit HPLC auf einer stereospezifischen Säule in die Komponenten getrennt, die anschließend mit einem UV-Detektor bei einer Wellenlänge von 238 nm analysiert werden.

Eine Milchsäureprobe wird direkt in der 3 millimolaren Kupfersulfatlösung gelöst und wie beschrieben mit HPLC analysiert.

### Restmonomergehalt im PLA

Eine abgewogene PLA-Probe wird in einem definierten Volumen von Chloroform gelöst. 100 µl dieser Lösung werden in ein GPC-System injiziert, das mit einer vernetzten Polystyrolsäure unterschiedlicher Porengröße und einem Brechungsindex-Detektor ausgerüstet ist. Als Laufmittel dient Chloroform.

Der Dilactid-Peak wird mit seiner Retentionszeit identifiziert. Aus seiner Fläche wird mit Hilfe einer zuvor aufgenommenen Kalibrierkurve die Substanzmenge bestimmt und mit der PLA-Einwaage in eine Dilactid-Konzentration umgerechnet.

### Beispiel 1

Dieses Beispiel zeigt die prinzipielle Eignung der Kombination von Hydrolyse und zyklisierender Depolymerisation zur Umwandlung von PLA in Rohlactid.

30 g PLA mit einer I.V. von 1,80 dl/g, entsprechend einer gewichtsmittleren Molmasse von 230.000, wird in einem Labor-Druckbehälter aus Edelstahl, der mit PTFE ausgekleidet ist, mit einer Wassermenge von 0,70 g versetzt und verschlossen. Der Behälter wird in einem Umluft-Trockenschrank eingebracht, der bei einer Temperatur von 190 °C gehalten wird. Nach 6 Stunden wird der Autoklav dem Trockenschrank entnommen und durch Stehenlassen bei Raumtemperatur abgekühlt. Der Behälter wird geöffnet, die entstandene zähe Masse entnommen und analysiert.

Durch Titration wird eine Carboxylendgruppenkonzentration von 1250 gefunden, die rechnerisch einer Molmasse von 800 Da entspricht.

20 g der zähen Masse werden in einem Dreihals-Glaskolben mit Rührer mit 0,184 mg Sn-Oktoat, gelöst in Toluol, versetzt. Nach Anlegen eines Vakuums von 5 mbar wird der Glaskolben von außen durch ein Ölbad auf 220 °C gebracht. Die Temperatur wird im Ölbad gemessen. Unter Rühren setzt die Entwicklung von Dämpfen ein, die aus dem Kolben abgeführt, in einem vertikalen Schlangenkühler unter Vakuum kondensiert und in einem gekühlten Glaskolben als gelbliche Kristalle gesammelt werden.

Die Depolymerisation wird nach 4,0 Stunden abgebrochen durch Entfernen des Dreihals-Glaskolbens aus dem beheizten Ölbad. Zu diesem Zeitpunkt ist die Dampfentwicklung zum Erliegen gekommen. Im Kolben bleibt ein schwarzbrauner Rückstand, von 1,8 g, der bei Raumtemperatur glasartig fest ist. Nach Brechen des Vakuums mit N₂ wird der Sammelkolben vom Kondensator genommen und der Inhalt gewogen und analysiert. Es finden sich 17,8 g eines Rohlactids mit einer Carboxylgruppenzahl von 350 mmol/kg. Die Probe enthält lt. HPLC-Analyse 95,2 % L-Lactid und 4,8 % meso-Lactid.

### Beispiel 2

Das Beispiel zeigt die Wirkung des erfindungsgemäßen Verfahrens. In einer Vorrichtung gemäß Figur 4 wird der Extruder 1 so eingestellt, dass er 1,5 kg/h zerkleinerte PLA-Abfälle einzieht und aufschmilzt. Die Abfälle zeigen eine mittlere I.V. von 1,64 dl/g (Chloroform, 30°C).

Das Druckhalteventil 8 am Ende der Rohrstrecke 2 wird auf 30 bar eingestellt. Die Temperatur der Schmelze am Austritt aus dem Extruder ist 210°C. Mit Hilfe der Dosierpumpe 5 werden 26,9 g/h demineralisiertes Wasser durch die Kapillare 4 in die Schmelze am Beginn der Rohrstrecke gedrückt. Eine Probe von der Schmelze hinter dem Druckhalteventil 8 zeigt eine Carboxylendgruppenkonzentration von 960 mmol/kg.

Die Dosierpumpe 7 fördert 300 ppm Sn als Sn-Oktoat über die Kapillare 6 in die hydrolysierte Schmelze am Ende der Rohrstrecke 2. Im Depolymerisationsreaktor 9 wird eine Schmelzetemperatur von 220°C eingestellt. Aus dem Reaktor werden 70 g/h an flüssigem Rückstand ausgetragen, aus der Kondensationsvorrichtung 13 1,410 kg/h Rohlactid. Am Rohlactid wird mittels HPLC eine Konzentration von 5,3 % meso-Lactid gemessen.

Eine Probe des Rohlactids von 2000 g ergibt nach Umkristallisation aus Toluol als Lösungsmittel sowie nach Abfiltrieren und Trocknen des Kristallisats 1300 g Reinlactid mit einer Carboxylgruppenkonzentration von 5 mmol/kg und einem Anteil an meso-Lactid von 0,4 %.

Die Reinlactidmenge wird in einem Laborautoklaven mit Rührer unter Stickstoff als Schutzgas aufgeschmolzen, mit 85 ppm Sn als Sn-Oktoat vermischt und nach Erhöhen der Schmelzetemperatur auf 180 °C 3 Stunden lang polymerisiert. Danach wird die PLA-Schmelze aus dem Rührbehälter als Strang ausgetragen, abgeschreckt und verfestigt durch Ziehen durch ein Wasserbad, und granuliert. Es werden 950 g Granulat gesammelt und durch Mischen homogenisiert. Der Restmonomergehalt, bestimmt mit Gelpermeationschromatografie, beträgt 5,2 %. Eine Probe des Granulats wird durch Lösen mit Chloroform und Fällen mit Isopropanol vom restlichen Monomer befreit und nach Trocknen analysiert. Die I.V. beträgt 1,82, der D-Anteil 0,5 %. Damit liegen die Eigenschaften des rezyklierten PLAs auf einem Niveau, das Neuware entspricht.

## Patentansprüche

1. Verfahren zur Rückgewinnung von Lactid aus Polylactid (PLA) oder Glycolid aus Polyglycolid (PGA), bei dem
a) PLA bzw. PGA in einem Extruder oder einem Schmelzrost aufgeschmolzen und in der Schmelze mit einem hydrolysierenden Medium in Kontakt gebracht und hydrolytisch zu PLA-Oligomeren mit einer zahlengemittelten Molmasse Mₙ zwischen 162 und 10.000 g/mol, gemessen nach der Beschreibung durch Säure-Base-Titration der Carboxylgruppen, bzw. zu PGA-Oligomeren mit einer zahlengemittelten Molmasse Mₙ zwischen 134 und 10.000 g/mol, gemessen nach der Beschreibung durch Säure-Base-Titration der Carboxylgruppen, abgebaut werden, wobei der hydrolytische Abbau in einer Hydrolysevorrichtung (2), die eine beheizbare Rohrstrecke aufweist, durchgeführt wird und
b) die PLA- bzw. PGA-Oligomeren anschließend einer zyklisierenden Depolymerisation zu Lactid bzw. Glycolid unterworfen werden, die in einem stromabwärts der Hydrolysevorrichtung (2) angeordneten Depolymerisationsreaktor (9), der als Umlaufverdampfer, Fallfilmverdampfer, Dünnschichtverdampfer oder als eine Kombination aus zwei oder drei dieser Bauarten ausgeführt ist, durchgeführt wird, wobei vor der zyklisierenden Depolymerisation in Stufe b) den Oligomeren ein Katalysator zugegeben wird.

2. Verfahren nach Anspruch 1, **dadurch gekennzeichnet, dass** das hydrolysierende Medium ausgewählt ist aus der Gruppe bestehend aus Wasser, Milchsäure, Glycolsäure, Mischungen aus Wasser und Milchsäure sowie Mischungen aus Wasser und Glycolsäure.

3. Verfahren nach einem der vorhergehenden Ansprüche, **dadurch gekennzeichnet, dass** in Schritt a) PLA bzw. PGA hydrolytisch zu PLA- bzw. PGA-Oligomeren mit einer zahlengemittelten Molmasse Mₙ zwischen 400 und 2.000 g/mol (gemessen durch Säure-Base-Titration der Carboxylgruppen) abgebaut wird.

4. Verfahren nach einem der vorhergehenden Ansprüche, **dadurch gekennzeichnet, dass** 50 mmol bis 10 mol, bevorzugt 100 mmol bis 5 mol, insbesondere 0,5 mol bis 3 mol an hydrolysierendem Medium pro 1 kg der Masse an PLA bzw. PGA zugegeben wird.

5. Verfahren nach einem der vorhergehenden Ansprüche, **dadurch gekennzeichnet, dass** während des hydrolytischen Abbaus in Stufe a)
α) eine Temperatur der Schmelze von 130 bis 300 °C, bevorzugt von 150 bis 250 °C, besonders bevorzugt von 190 und 230 °C eingestellt wird,
β) ein Druck von 5 bis 500 bar, bevorzugt von 10 bis 300 bar, besonders bevorzugt von 20 bis 200 bar eingestellt wird und/oder
γ) eine Verweilzeit der Schmelze von 0,1 bis 50 min, bevorzugt von 1 bis 15 min, besonders bevorzugt von 1 bis 5 min eingehalten wird.

6. Verfahren nach einem der vorhergehenden Ansprüche, **dadurch gekennzeichnet, dass** der Katalysator in einer Konzentration von 0,01 bis 50 mmol/kg Oligomere, bevorzugt von 0,1 bis 10 mmol/ kg Oligomere zugegeben wird.

7. Verfahren nach einem der vorhergehenden Ansprüche, **dadurch gekennzeichnet, dass** das PLA bzw. das PGA zumindest teilweise oder ganz aus Abfallmaterial stammt, wobei die PLA- bzw. PGA-Abfälle vor Aufgabe in Stufe a)
α) durch Sortieren, Waschen und andere mechanische Trennmethoden von Fremdstoffen gereinigt werden, so dass bevorzugt der Anteil an Fremdstoffen und/oder Verunreinigungen weniger als 5 Gew.-%, weiter bevorzugt weniger als 1 Gew.-%, bezogen auf die Masse an PLA bzw. PGA beträgt, und/oder
(3) zerkleinert werden, so dass die maximale Abmessung der erhaltenen zerkleinerten Abfälle 15 mm beträgt.

8. Verfahren nach einem der vorhergehenden Ansprüche, **dadurch gekennzeichnet, dass** die in Stufe a) erhaltenen Oligomeren
α) zu Milchsäure bzw. Glycolsäure, die in eine Polykondensationsstufe aufgegeben wird, wobei aus Milchsäure bzw. Glycolsäure deren jeweilige Oligomere hergestellt werden,
(β) in eine Polykondensationsstufe von Milchsäure bzw. Glycolsäure, wobei aus Milchsäure bzw. Glycolsäure deren jeweilige Oligomere hergestellt werden, und/oder
γ) zu den aus einer Polykondensationsstufe von Milchsäure bzw. Glycolsäure erhaltenen Oligomeren,
zugegeben werden und das so erhaltene Oligomerengemisch der zyklisierenden Depolymerisation (Stufe b)) zugeführt wird.

9. Verfahren nach einem der vorhergehenden Ansprüche, **dadurch gekennzeichnet, dass** das durch die zyklisierende Depolymerisation (Stufe b)) erhaltene Lactid bzw. Glycolid mit Lactid bzw. Glycolid, das durch Polykondensation von Milchsäure bzw. Glycolsäure zu Oligomeren und zyklisierender Depolymerisation dieser Oligomere erhalten wurde, vermischt wird.

10. Verfahren nach einem der vorhergehenden Ansprüche, **dadurch gekennzeichnet, dass** das nach Stufe b) erhaltene Lactid in eine verglichen mit dem unmittelbar aus Stufe b) erhaltenen Lactid
α) an meso-Lactid abgereicherte Fraktion und eine an meso-Lactid angereicherte Fraktion aufgetrennt wird, oder
(β) in eine an L-Lactid angereicherte Fraktion, eine an D-Lactid angereicherte Fraktion und/oder eine an meso-Lactid angereicherte Fraktion aufgetrennt wird, wobei die Konzentration dieser Komponenten in der Fraktion ≥ 50 Gew.-%, bevorzugt ≥ 90 Gew.-%, besonders bevorzugt ≥ 98 Gew.-% beträgt.

11. Verfahren nach einem der vorhergehenden Ansprüche, **dadurch gekennzeichnet, dass**
α) die Oligomeren vor Schritt b) und/oder
β) das erhaltene Lactid bzw. Glycolid nach Schritt b)
einer Reinigung, insbesondere einer destillativen Reinigung und/oder einer Umkristallisation unterzogen wird/werden.

12. Verfahren nach einem der vorhergehenden Ansprüche, **dadurch gekennzeichnet, dass** das PLA bzw. PGA in die Stufe a) in aufgeschmolzenem Zustand aufgegeben wird oder während Stufe a) aufgeschmolzen wird.

13. Verfahren nach einem der vorhergehenden Ansprüche, **dadurch gekennzeichnet, dass** das Verfahren kontinuierlich durchgeführt wird.

14. Verfahren zur Herstellung von PLA bzw. PGA, bei dem Lactid bzw. Glycolid in einer Ringöffnungspolymerisation zu PLA bzw. PGA umgesetzt wird,
**dadurch gekennzeichnet,**
**dass** zunächst ein Rückgewinnungsverfahren nach einem der vorhergehenden Ansprüche, durchgeführt wird und zumindest ein Teil oder die Gesamtheit des für die Ringöffnungspolymerisation eingesetzten Lactids bzw. Glycolids nach dem Verfahren zur Rückgewinnung nach einem der vorhergehenden Ansprüche hergestellt wurde.

15. Vorrichtung zur kontinuierlichen Rückgewinnung von Lactid aus PLA oder Glycolid aus PGA, umfassend
a) eine Vorrichtung (1) zum Aufschmelzen von PLA bzw. PGA und/oder eine Vorrichtung zur Zuführung einer PLA- bzw. PGA-Schmelze, wobei die Vorrichtung (1) zum Aufschmelzen ein Extruder oder ein Schmelzrost ist,
b) eine stromabwärts der Vorrichtung (1) zum Aufschmelzen und/oder der Vorrichtung zur Zuführung einer PLA- bzw. PGA-Schmelze angeordnete Hydrolysevorrichtung (2) zur Durchführung einer partiellen Hydrolyse der PLA- bzw. PGA-Schmelzen zu PLA- bzw. PGA-Oligomeren, wobei die Hydrolysevorrichtung (2) eine beheizbare Rohrstrecke aufweist, sowie
c) einen stromabwärts der Hydrolysevorrichtung (2) angeordneten Depolymerisationsreaktor (9), der als Umlaufverdampfer, Fallfilmverdampfer, Dünnschichtverdampfer oder als eine Kombination aus zwei oder drei dieser Bauarten ausgeführt ist.

16. Vorrichtung nach vorhergehendem Anspruch, **dadurch gekennzeichnet, dass** die Hydrolysevorrichtung (2) mit der Vorrichtung (1) zum Aufschmelzen und/oder der Vorrichtung zur Zuführung einer PLA- bzw. PGA-Schmelze über eine Schmelzeleitung für die Schmelze in Verbindung steht.

17. Vorrichtung nach einem der beiden vorhergehenden Ansprüche, **dadurch gekennzeichnet, dass** die Vorrichtung zur Zuführung einer PLA- bzw. PGA-Schmelze eine Schmelzeleitung und/oder eine Schmelzepumpe ist, und/oder die beheizbare Rohrstrecke der Hydrolysevorrichtung (2) beheizbare oder unbeheizbare statische Mischelemente (3) aufweist.

18. Vorrichtung nach vorhergehendem Anspruch, **dadurch gekennzeichnet, dass** die Hydrolysevorrichtung (2) über
α) eine Eintragsmöglichkeit (4) für ein hydrolysierendes Medium, die bevorzugt in die Schmelzezuführung der Hydrolysevorrichtung (2) mündet, und/oder
β) eine Eintragsmöglichkeit (6) für einen Depolymerisationskatalysator, die bevorzugt in die Schmelzeabführung der Hydrolysevorrichtung (2) mündet,
verfügt.

19. Vorrichtung nach einem der beiden vorhergehenden Ansprüche, **dadurch gekennzeichnet, dass** der Depolymerisationsreaktor (9) eine Abzugsvorrichtung (10) aufweist, über die nicht umgesetzte Schmelze ausgetragen werden kann.

20. Vorrichtung nach einem der Ansprüche 15 bis 19, **dadurch gekennzeichnet, dass** der Depolymerisationsreaktor (9) einen kopfseitig angeordneten Abzug für Lactid- bzw. Glycoliddämpfe aufweist, der in eine Kondensationsvorrichtung (12) für Lactid- bzw. Glycoliddämpfe mündet.

21. Vorrichtung nach vorhergehendem Anspruch, **dadurch gekennzeichnet, dass** der Kondensationsvorrichtung (12) eine Bevorratungsvorrichtung (11) für Lactid bzw. Glycolid und/oder eine Reinigungsvorrichtung für Lactid bzw. Glycolid nachgeschaltet ist.

22. Vorrichtung nach einem der Ansprüche 16 bis 21, **dadurch gekennzeichnet, dass** zwischen der Hydrolysevorrichtung (2) und dem Depolymerisationsreaktor (9) ein Polykondensationsreaktor zur Herstellung von PLA- bzw. PGA-Oligomeren durch Polykondensation von Milchsäure bzw. Glycolsäure angeordnet ist, wobei der Auslass der Hydrolysevorrichtung (2) vor, in oder nach dem Polykondensationsreaktor mündet.

23. Vorrichtung nach einem der Ansprüche 15 bis 22, **dadurch gekennzeichnet, dass** der Auslass des Depolymerisationsreaktors (9) mit dem Auslass eines Reaktors, der in einer ersten Stufe mittels Polykondensation von Milchsäure bzw. Glycolsäure PLA- bzw. PGA-Oligomere und in einer zweiten Stufe aus diesen PLA- bzw. PGA-Oligomere mittels zyklisierender Depolymerisation Lactid bzw. Gycolid erzeugt, zusammenläuft.

24. Vorrichtung zur Herstellung von PLA bzw. PGA durch Ringöffnungspolymerisation von Lactid bzw. Glycolid, umfassend eine Vorrichtung zur kontinuierlichen Rückgewinnung von Lactid aus PLA-Abfällen oder Glycolid aus PGA-Abfällen nach einem der Ansprüche 15 bis 23 sowie nachgeschaltet eine Ringöffnungspolymerisationsvorrichtung.

## Claims

1. Process for recovering lactide from polylactide (PLA) or glycolide from polyglycolide (PGA), in which
a) PLA or PGA is melted in an extruder or a melting grid and is brought into contact with a hydrolyzing medium in the melt and is hydrolytically degraded to PLA oligomers having a number-average molecular weight Mₙ of between 162 and 10,000 g/mol, measured according to the specification by acid-base titration of the carboxyl groups, or to PGA oligomers having a number-average molecular weight Mₙ of between 134 and 10,000 g/mol, measured according to the specification by acid-base titration of the carboxyl groups, the hydrolytic degradation being carried out in a hydrolysis device (2) which has a heatable tubular section, and
b) the PLA or PGA oligomers are then subjected to cyclizing depolymerization to lactide or glycolide, which is carried out in a depolymerization reactor (9) which is arranged downstream of the hydrolysis device (2) and is designed as a circulating evaporator, falling-film evaporator, thin-film evaporator or as a combination of two or three of these designs, a catalyst being added to the oligomers prior to the cyclizing depolymerization in stage b).

2. Process according to claim 1, **characterized in that** the hydrolyzing medium is selected from the group consisting of water, lactic acid, glycolic acid, mixtures of water and lactic acid and mixtures of water and glycolic acid.

3. Process according to one of the preceding claims, **characterized in that** in step a) PLA or PGA is hydrolytically degraded to PLA or PGA oligomers having a number-average molecular weight Mₙ of between 400 and 2,000 g/mol (measured by acid-base titration of the carboxyl groups).

4. Process according to one of the preceding claims, **characterized in that** 50 mmol to 10 mol, preferably 100 mmol to 5 mol, in particular 0.5 mol to 3 mol of hydrolyzing medium is added per 1 kg of the mass of PLA or PGA.

5. Process according to one of the preceding claims, **characterized in that** during the hydrolytic degradation in stage a)
α) a melt temperature of 130 to 300 °C, preferably 150 to 250 °C, particularly preferably 190 and 230 °C is set,
β) a pressure of 5 to 500 bar, preferably 10 to 300 bar, particularly preferably 20 to 200 bar is set and/or
γ) a dwell time of the melt of 0.1 to 50 min, preferably of 1 to 15 min, particularly preferably of 1 to 5 min is maintained.

6. Process according to one of the preceding claims, **characterized in that** the catalyst is added in a concentration of 0.01 to 50 mmol/kg oligomers, preferably from 0.1 to 10 mmol/kg oligomers.

7. Process according to one of the preceding claims, **characterized in that** the PLA or the PGA originates at least partly or entirely from waste material, the PLA or PGA waste, prior to being subjected to stage a),
α) being cleaned of foreign matter by sorting, washing and other mechanical separation methods, so that preferably the proportion of foreign matter and/or impurities is less than 5% by weight, more preferably less than 1% by weight, in relation to the mass of PLA or PGA, and/or
β) are comminuted, so that the maximum dimension of the comminuted waste obtained is 15 mm.

8. Process according to one of the preceding claims, **characterized in that** the oligomers obtained in stage a) are added to:
α) lactic acid or glycolic acid, which is subjected to a polycondensation stage, whereby their respective oligomers are produced from lactic acid or glycolic acid,
β) a polycondensation stage of lactic acid or glycolic acid, whereby their respective oligomers are produced from lactic acid or glycolic acid, and/or
γ) the oligomers obtained from a polycondensation stage of lactic acid or glycolic acid,
and the resultant oligomer mixture is subjected to the cyclizing depolymerization (stage b)).

9. Process according to one of the preceding claims, **characterized in that** the lactide or glycolide obtained by the cyclizing depolymerization (stage b)) is mixed with lactide or glycolide obtained by polycondensation of lactic acid or glycolic acid to oligomers and cyclizing depolymerization of these oligomers.

10. Process according to one of the preceding claims, **characterized in that** the lactide obtained after stage b), compared with the lactide obtained directly from stage b),
α) is separated into a fraction depleted in meso-lactide and a fraction enriched in meso-lactide, or
β) is separated into a fraction enriched in L-lactide, a fraction enriched in D-lactide and/or a fraction enriched in meso-lactide, the concentration of these components in the fraction being ≥ 50 wt.%, preferably ≥ 90 wt.%, particularly preferably > 98 wt.%.

11. Process according to one of the preceding claims, **characterized in that**
α) the oligomers, prior to step b) and/or
β) the lactide or glycolide obtained after step b)
is/are subjected to purification, in particular distillative purification and/or recrystallization.

12. Process according to one of the preceding claims, **characterized in that** the PLA or PGA is subjected to stage a) in the molten state or is melted during stage a).

13. Process according to one of the preceding claims, **characterized in that** the process is carried out continuously.

14. Process for the production of PLA or PGA, in which lactide or glycolide is converted to PLA or PGA in a ring-opening polymerization,
**characterized in that**
a recovery process according to one of the preceding claims is first carried out and at least part or all of the lactide or glycolide used for the ring-opening polymerization has been produced according to the recovery process according to one of the preceding claims.

15. Apparatus for the continuous recovery of lactide from PLA or glycolide from PGA, comprising
a) a device (1) for melting PLA or PGA and/or an device for supplying a PLA or PGA melt, wherein the device (1) for melting is an extruder or a melting grid,
b) a hydrolysis device (2) arranged downstream of the device (1) for melting and/or the device for supplying a PLA or PGA melt, for carrying out a partial hydrolysis of the PLA or PGA melts to PLA or PGA oligomers, the hydrolysis device (2) having a heatable tubular section, and
c) a depolymerization reactor (9) arranged downstream of the hydrolysis device (2), which is designed as a circulating evaporator, falling-film evaporator, thin-film evaporator or as a combination of two or three of these designs.

16. Device according to the preceding claim, **characterized in that** the hydrolysis device (2) is connected to the device (1) for melting and/or the device for supplying a PLA or PGA melt via a melt line for the melt.

17. Device according to one of the two preceding claims, **characterized in that** the device for supplying a PLA or PGA melt is a melt line and/or a melt pump, and/or the heatable tubular section of the hydrolysis device (2) has heatable or unheatable static mixing elements (3).

18. Device according to the preceding claim, **characterized in that** the hydrolysis device (2) comprises
α) an inlet opening (4) for a hydrolyzing medium, which preferably opens into the melt supply line of the hydrolysis device (2), and/or
β) an inlet opening (6) for a depolymerization catalyst, which preferably opens into the melt discharge line of the hydrolysis device (2).

19. Device according to one of the two preceding claims, **characterized in that** the depolymerization reactor (9) has a discharge device (10) via which unconverted melt can be discharged.

20. Process according to one of claims 15 to 19, **characterized in that** the depolymerization reactor (9) has an outlet for lactide or glycolide vapors arranged on the head side, which opens into a condensation device (12) for lactide or glycolide vapors.

21. Device according to the preceding claim, **characterized in that** a storage device (11) for lactide or glycolide and/or a purification device for lactide or glycolide is arranged downstream of the condensation device (12).

22. Process according to one of claims 16 to 21, **characterized in that** a polycondensation reactor for the production of PLA or PGA oligomers by polycondensation of lactic acid or glycolic acid is arranged between the hydrolysis device (2) and the depolymerization reactor (9), the outlet of the hydrolysis device (2) opening before, in or after the polycondensation reactor.

23. Process according to one of claims 15 to 22, **characterized in that** the outlet of the depolymerization reactor (9) merges with the outlet of a reactor which in a first stage produces PLA or PGA oligomers by means of polycondensation of lactic acid or glycolic acid and in a second stage produces lactide or glycolide from these PLA or PGA oligomers by means of cyclizing depolymerization.

24. Device for producing PLA or PGA by ring-opening polymerization of lactide or glycolide, comprising a device for the continuous recovery of lactide from PLA waste or glycolide from PGA waste according to one of claims 15 to 23 and, arranged downstream thereof, a ring-opening polymerization device.

## Revendications

1. Procédé pour le recyclage d'un lactide à partir d'un polylactide (PLA) ou d'un glycolide à partir d'un polyglycolide (PGA), dans lequel
a) le PLA respectivement le PGA est fondu dans une extrudeuse ou une grille de fusion et sont mis en contact, dans la matière fondue, avec un milieu hydrolysant et est décomposé par hydrolyse en oligomères de PLA avec une masse molaire moyenne Mₙ entre 162 et 10 000 g/mol, mesurée selon la description par titration acide-base des groupes carboxyles, respectivement en oligomères de PGA avec une masse molaire moyenne Mₙ entre 134 et 10 000 g/mol, mesurée selon la description par titration acide-base des groupes carboxyles, dans lequel la décomposition par hydrolyse est effectuée dans un dispositif d'hydrolyse (2) qui comprend une section de tube pouvant être chauffée et
b) les oligomères de PLA respectivement de PGA sont ensuite soumis à une dépolymérisation par cyclisation en lactide respectivement en glycolide, qui est effectuée dans un réacteur de dépolymérisation (9) disposé en aval du dispositif d'hydrolyse (2), qui est conçu comme un évaporateur à circulation, un évaporateur à film descendant, un évaporateur à couche mince ou une combinaison de deux ou trois de ces types de conception, dans lequel, avant la dépolymérisation par cyclisation, un catalyseur est ajouté aux oligomères à l'étape b).

2. Procédé selon la revendication 1, **caractérisé en ce que** le milieu hydrolysant est sélectionné dans le groupe constitué d'eau, d'acide lactique, d'acide glycolique, de mélanges d'eau et d'acide lactique ainsi que de mélanges d'eau et d'acide glycolique.

3. Procédé selon l'une des revendications précédentes, **caractérisé en ce que**, à l'étape a), le PLA respectivement le PGA est décomposé par hydrolyse en oligomères de PLA respectivement de PGA avec une masse molaire moyenne Mₙ entre 400 et 2 000 g/mol (mesurée par titration acide-base des groupes carboxyles).

4. Procédé selon l'une des revendications précédentes, **caractérisé en ce que** 50 mmol à 10 mol, de préférence de 100 mmol à 5 mol, plus particulièrement 0,5 mol à 3 mol de milieu hydrolysant sont ajoutées pour 1 kg de masse de PLA respectivement de PGA.

5. Procédé selon l'une des revendications précédentes, **caractérisé en ce que**, pendant la décomposition par hydrolyse à l'étape a),
α) une température de la matière fondue est ajustée entre 130 et 300 °C, de préférence entre 150 et 250 °C, plus particulièrement de préférence entre 190 et 230 °C,
β) une pression est ajustée entre 5 et 500 bar, de préférence entre 10 et 300 bar, plus particulièrement de préférence entre 20 et 200 bar et/ou
γ) un temps de séjour de la matière fondue entre 0,1 et 50 min, de préférence entre 1 et 15 min, plus particulièrement de préférence entre 1 et 5 min est respecté.

6. Procédé selon l'une des revendications précédentes, **caractérisé en ce que** le catalyseur est ajouté avec une concentration de 0,01 à 50 mmol/kg d'oligomères, de préférence de 0,1 à 10 mmol/kg d'oligomères.

7. Procédé selon l'une des revendications précédentes, **caractérisé en ce que** le PLA respectivement le PGA provient au moins partiellement ou entièrement d'un matériau de déchet, dans lequel les déchets de PLA respectivement de PGA, avant l'ajout à l'étape a),
α) sont nettoyés des matières étrangères par tri, lavage et d'autres procédés de séparation mécanique, de sorte que, de préférence, la part des matières étrangères et/ou des impuretés représente moins de 5 % en poids, de préférence moins de 1 % en poids, par rapport à la masse de PLA respectivement de PGA et/ou
β) sont broyés, de sorte que la dimension maximale des déchets broyés obtenus représente 15 mm.

8. Procédé selon l'une des revendications précédentes, **caractérisé en ce que** les oligomères obtenus à l'étape a)
α) sont ajoutés à de l'acide lactique respectivement de l'acide glycolique, qui est versé dans une étape de polycondensation, dans lequel, à partir de l'acide lactique respectivement de l'acide glycolique, leurs oligomères respectifs sont formés,
β) sont ajoutés dans une étape de polycondensation de l'acide lactique respectivement de l'acide glycolique, dans lequel, à partir de l'acide lactique respectivement de l'acide glycolique, leurs oligomères respectifs sont formés et/ou
γ) sont ajoutés aux oligomères obtenus à partir d'une étape de polycondensation de l'acide lactique respectivement de l'acide glycolique,
et le mélange d'oligomères ainsi obtenu est ajouté à la dépolymérisation de cyclisation (étape b)).

9. Procédé selon l'une des revendications précédentes, **caractérisé en ce que** le lactide respectivement le glycolide obtenu grâce à la dépolymérisation de cyclisation (étape b)) est mélangé au lactide respectivement au glycolide qui a été obtenu par la polycondensation de l'acide lactique respectivement de l'acide glycolique en oligomères et par la dépolymérisation de cyclisation de ces oligomères.

10. Procédé selon l'une des revendications précédentes, **caractérisé en ce que** le lactide obtenu après l'étape b) est séparée en une fraction, par rapport au lactide obtenu directement à partir de l'étape b),
α) appauvrie en méso-lactide et une fraction enrichie en méso-lactide ou
β) enrichie en L-lactide, une fraction enrichie en D-lactide et/ou une fraction enrichie en méso-lactide, dans lequel la concentration de ces composants dans la fraction représente ≥ 50 % en poids, de préférence ≥ 90 % en poids, plus particulièrement de préférence ≥ 98 % en poids.

11. Procédé selon l'une des revendications précédentes, **caractérisé en ce que**
α) les oligomères sont soumis, avant l'étape b) et/ou
β) le lactide respectivement le glycolide obtenu est soumis, après l'étape b),
à une purification, plus particulièrement à une purification par distillation et/ou à une recristallisation.

12. Procédé selon l'une des revendications précédentes, **caractérisé en ce que** le PLA respectivement le PGA est ajouté, dans l'étape a), dans un état fondu ou est fondu pendant l'étape a).

13. Procédé selon l'une des revendications précédentes, **caractérisé en ce que** le procédé est exécuté de manière continue.

14. Procédé de fabrication de PLA respectivement de PGA, dans lequel un lactide respectivement un glycolide est converti, dans une polymérisation par ouverture de cycle, en PLA respectivement PGA,
**caractérisé en ce que**
un procédé de recyclage selon l'une des revendications précédentes est d'abord exécuté et au moins une partie ou la totalité du lactide respectivement du glycolide utilisé pour la polymérisation par ouverture de cycle a été fabriquée selon le procédé de recyclage selon l'une des revendications précédentes.

15. Dispositif pour le recyclage en continu de lactide à partir de PLA ou de glycolide à partir de PGA, comprenant
a) un dispositif (1) pour la fusion de PLA respectivement de PGA et/ou un dispositif d'introduction d'une fonte de PLA respectivement de PGA, dans lequel le dispositif (1) de fusion est une extrudeuse ou une grille de fusion,
b) un dispositif d'hydrolyse (2) disposé en aval du dispositif (1) pour la fusion et/ou du dispositif pour l'introduction d'une fonte de PLA respectivement de PGA, pour la réalisation d'une hydrolyse partielle des fontes de PLA respectivement de PGA en oligomères de PLA respectivement de PGA, dans lequel le dispositif d'hydrolyse (2) comprend une section de tube pouvant être chauffée ainsi que
c) un réacteur de dépolymérisation (9) disposé en aval du dispositif d'hydrolyse (2), qui est conçu comme un évaporateur à circulation, un évaporateur à film descendant, un évaporateur à couche mince ou comme une combinaison de deux ou trois de ces types de conception.

16. Dispositif selon la revendication précédente, **caractérisé en ce que** le dispositif d'hydrolyse (2) est relié avec le dispositif (1) de fusion et/ou le dispositif d'introduction d'une fonte de PLA respectivement de PGA par l'intermédiaire d'une conduite pour la matière fondue.

17. Dispositif selon l'une des deux revendications précédentes, **caractérisé en ce que** le dispositif d'introduction d'une fonte de PLA respectivement de PGA est une conduite de matière fondue et/ou une pompe de matière fondue et/ou la section de tube pouvant être chauffée du dispositif d'hydrolyse (2) comprend des éléments de mélange statiques (3) pouvant être chauffés ou non.

18. Dispositif selon la revendication précédente, **caractérisé en ce que** le dispositif d'hydrolyse (2) est muni
α) d'une possibilité d'introduction (4) pour un milieu hydrolysant, qui débouche de préférence dans l'alimentation en matière fondue du dispositif d'hydrolyse (2) et/ou
β) une possibilité d'introduction (6) pour un catalyseur de dépolymérisation, qui débouche de préférence dans l'évacuation de matière fondue du dispositif d'hydrolyse (2).

19. Dispositif selon l'une des deux revendications précédentes, **caractérisé en ce que** le réacteur de dépolymérisation (9) comprend un dispositif d'extraction (10) par l'intermédiaire duquel la matière fondue non transformée peut être évacuée.

20. Dispositif selon l'une des revendications 15 à 19, **caractérisé en ce que** le réacteur de dépolymérisation (9) comprend une évacuation disposée côté tête, pour les vapeurs de lactide respectivement de glycolide, qui débouche dans un dispositif de condensation (12) pour les vapeurs de lactide respectivement de glycolide.

21. Dispositif selon la revendication précédente, **caractérisé en ce que**, en aval du dispositif de condensation (12), est disposé un dispositif d'approvisionnement (11) en lactide respectivement en glycolide et/ou un dispositif de nettoyage pour le lactide respectivement le glycolide.

22. Dispositif selon l'une des revendications 16 à 21, **caractérisé en ce que**, entre le dispositif d'hydrolyse (2) et le réacteur de dépolymérisation (9), est disposé un réacteur de polycondensation pour la fabrication d'oligomères de PLA respectivement de PGA par polycondensation d'acide lactique respectivement d'acide glycolique, dans lequel la sortie du dispositif d'hydrolyse (2) débouche avant, dans ou après le réacteur de polycondensation.

23. Dispositif selon l'une des revendications 15 à 22, **caractérisé en ce que** la sortie du réacteur de dépolymérisation (9) converge avec la sortie d'un réacteur qui produit, dans une première étape, par polycondensation d'acide lactique respectivement d'acide glycolique, des oligomères de PLA respectivement de PGA et, dans une deuxième étape, à partir de ces oligomères de PLA respectivement de PGA, par dépolymérisation par cyclisation, un lactide respectivement un glycolide.

24. Dispositif pour la fabrication de PLA respectivement de PGA, par polymérisation par ouverture de cycle, de lactide respectivement de glycolide, comprenant un dispositif pour la récupération en continu de lactide à partir de déchets de PLA ou de glycolide à partir de déchets de PGA selon l'une des revendications 15 à 23 et, en aval, un dispositif de polymérisation par ouverture de cycle.
